(19) Europäisches Patentamt
European Patent Office
Office européen des brevets

(11) EP 0 654 460 B1

(12) **FASCICULE DE BREVET EUROPEEN**

(45) Date de publication et mention de la délivrance du brevet: **18.06.1997 Bulletin 1997/25**

(51) Int. Cl.[6]: **C07C 49/395**, C07C 45/68, C07C 49/697, C07C 49/04

(21) Numéro de dépôt: **94402556.8**

(22) Date de dépôt: **14.11.1994**

(54) **Procédé de C-alkylation d'un compose cétonique**
Verfahren zur C-Alkylierung einer ketonischen Verbindung
Process for the C-alkylation of a ketonic compound

(84) Etats contractants désignés:
**AT BE CH DE FR GB IE IT LI NL**

(30) Priorité: **22.11.1993 FR 9313920**

(43) Date de publication de la demande:
**24.05.1995 Bulletin 1995/21**

(73) Titulaire: **RHONE-POULENC CHIMIE**
**92408 Courbevoie Cédex (FR)**

(72) Inventeurs:
- **Spagnol, Michel**
**F-69003 Lyon (FR)**
- **Gilbert, Laurent**
**F-69007 Lyon (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 166 490**
**EP-A- 0 194 591**
**EP-A- 0 240 863**
**EP-A- 0 378 953**
**GB-A- 1 448 855**
**US-A- 2 549 520**
**US-A- 3 188 350**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 654 460 B1

Printed by Rank Xerox (UK) Business Services
2.14.8/3.4

## Description

La présente invention a pour objet un procédé de C-alkylation d'un composé cétonique.

Plus précisément, l'invention concerne un procédé de C-alkylation d'un composé cétonique acyclique ou cyclique présentant au moins un atome d'hydrogène ou une fonction ester en position $\alpha$ par rapport au groupe carbonyle.

Dans son mode préféré, l'invention a trait à un procédé de C-alkylation de la cyclopentanone, de la 2-méthylcyclopentanone et des cyclopentanones substituées.

Il existe dans la littérature, des travaux décrivant des procédés d'alkylation d'une cétone : l'alkylation s'effectuant sur l'atome d'hydrogène en position $\alpha$ par rapport au groupe carbonyle.

Les procédés classiques consistent à transformer la cétone en énolate, par traitement à l'aide d'une base, puis réaction de l'énolate obtenu avec un agent d'alkylation. Une illustration de cette technique est donnée par la publication de Jean d'ANGELO [Tetrahedron, 32, pp. 2979-2990 (1976)]. L'énolate est formé par réaction de la cétone, avec une base telle que $iPr_2NLi$, à basse température (-78°C), dans un solvant aprotique polaire, par exemple le THF.

Ce procédé n'est pas extrapolable à l'échelle industrielle et la présence d'une base entraîne la formation de sels qu'il faut ensuite éliminer.

Un autre procédé décrit par G. STORCK et coll. [J. Am. Chem. Soc.,85, pp. 207-216 (1963)] réside dans l'alkylation des composés carbonylés, par passage par l'intermédiaire d'une énamine qui réagit avec l'agent d'alkylation conduisant alors à une imine qui est soumise à une étape ultérieure d'hydrolyse. Ce procédé d'alkylation est très complexe, en raison des nombreuses étapes qui le composent.

Il a par ailleurs été décrit par Z. JEDLINSKI et coll. [Synlett, pp. 213, (1990)], une méthode d'alkylation toute aussi compliquée. Elle passe par l'intermédiaire d'un énolate par réaction de la cétone cyclique avec une base qui est un complexe de potassium et d'éther couronne 18-C-6, dans le THF. L'énolate de cétone obtenu réagit avec l'agent d'alkylation conduisant ainsi à une cétone $\alpha$ -alkylée.

Tous les procédés connus dans l'état de la technique souffrent d'un inconvénient qui est la difficulté de les mettre en oeuvre industriellement.

La présente invention permet de pallier aux inconvénients précités en proposant un procédé simple et industriel d'alkylation d'un composé cétonique.

Plus précisément, la présente invention a pour objet un procédé de C-alkylation d'un composé cétonique ayant au moins un atome d'hydrogène ou une fonction ester en position $\alpha$ par rapport au groupe carbonyle caractérisé par le fait que l'on conduit la réaction d'alkylation en position $\alpha$ par rapport au groupe carbonyle, en présence d'une quantité efficace d'un catalyseur comprenant un anion orthophosphate condensé ou non.

Dans l'exposé qui suit de la présente invention, on entend par "composé cétonique", un composé hydrocarboné acyclique ou cyclique comprenant au moins un groupe carbonyle et ayant au moins un atome d'hydrogène ou une fonction ester en position $\alpha$ par rapport au groupe carbonyle.

Le nombre de groupe carbonyle présent dans le substrat de départ peut donc être supérieur à 1, par exemple égal à 2 et l'on est alors en présence d'une dione.

Une première variante d'exécution de l'invention consiste à faire réagir un composé cétonique présentant au moins un atome d'hydrogène en position $\alpha$ du groupe carbonyle avec un agent d'alkylation.

Une autre variante de l'invention réside dans la mise en oeuvre d'un composé cétonique présentant au moins une fonction ester en position $\alpha$ par rapport au groupe carbonyle. Dans un tel cas, l'agent d'alkylation n'est pas nécessairement apporté par voie extérieure mais peut être fourni par la fonction ester.

Par "fonction ester", on entend un radical de type $-COOR_e$ dans lequel $R_e$ est le radical alkylant qui va se fixer en position $\alpha$ par rapport au groupe carbonyle. Il peut être d'une nature quelconque et est choisi en fonction du groupe que l'on souhaite introduire. D'une manière préférée, le radical $R_e$ est un radical alkyle linéaire ou ramifié ayant de 1 à 6 atomes de carbone, et de préférence, de 1 à 4 atomes de carbone. $R_e$ représente préférentiellement un radical méthyle ou éthyle.

Le procédé de l'invention s'applique à tout composé cétonique qu'il soit cyclique ou non cyclique.

Il convient particulièrement bien à toute cétone cyclique, présentant un nombre de groupe carbonyle présent sur un cycle qui peut être supérieur à 1.

Etant donné que la cétone cyclique de départ mise en oeuvre dans le procédé de l'invention peut être polycyclique, notamment bicyclique, il s'en suit que le nombre de groupes carbonyle dans le composé cétonique de départ peut être égal à 3, voire même à 4 ou plus.

La cétone cyclique mise en oeuvre dans le procédé de l'invention peut donc être une cétone mono- ou polycarbonylée. Il peut s'agir d'une cétone monocyclique ou polycyclique.

Le procédé de l'invention convient tout à fait bien aux cétones cycliques répondant à la formule générale (Ia):

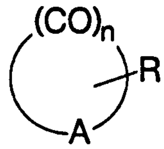

(Ia)

dans laquelle:

- A symbolise le reste d'un cycle formant tout ou partie d'un système monocyclique ou polycyclique comprenant au moins un groupe carbonyle et ayant au moins un atome d'hydrogène ou une fonction ester en position $\alpha$ par rapport au groupe carbonyle,
- R représente un atome d'hydrogène ou un ou plusieurs substituants, identiques ou différents,
- n est un nombre de préférence égal à 1 ou 2.

On précisera, sans pour autant limiter la portée de l'invention, que le reste A éventuellement substitué représente préférentiellement, le reste :

- d'un composé monocyclique, carbocyclique, saturé ou insaturé,
- d'un composé polycyclique comprenant au moins deux carbocycles, saturés et/ou insaturés,
- d'un composé polycyclique comprenant au moins deux cycles saturés et/ou insaturés : un ou plusieurs des atomes de carbone pouvant être remplacés par un hétéroatome,
- d'un composé polycyclique comprenant au moins deux carbocycles dont l'un d'eux est aromatique.

La cétone cyclique de formule (Ia) peut donc être un composé monocyclique ou polycyclique.

Lorsqu'il s'agit d'un composé monocyclique, le nombre d'atomes de carbone dans le cycle peut varier largement de 3 à 20 atomes de carbone mais il est de préférence de 5 ou 6 atomes de carbone.

Le carbocycle peut être saturé ou comprenant 1 ou 2 insaturations dans le cycle, de préférence de 1 à 2 doubles liaisons qui sont le plus souvent en position $\alpha$ du groupe carbonyle.

Le composé peut être également polycyclique, de préférence bicyclique ce qui signifie qu'au moins deux cycles ont deux atomes de carbone en commun.

Dans le cas des composés polycycliques, la condensation en carbone de chaque cycle est plus faible, généralement de 3 à 8 mais est égale, de préférence à 5 ou 6 atomes de carbone.

Le composé polycyclique peut comprendre au moins deux cycles saturés et/ou insaturés : un ou plusieurs (de préférence deux) des atomes de carbone pouvant être remplacés par un hétéroatome, de préférence un atome d'oxygène ou d'azote.

Le composé polycyclique peut comprendre au moins deux carbocycles dont l'un d'eux est aromatique, le cycle aromatique étant de préférence un cycle benzénique.

La cétone cyclique de formule (Ia) peut être porteuse d'un ou plusieurs substituants.

Le nombre de substituants présents sur le cycle dépend de la condensation en carbone du cycle et de la présence ou non d'insaturations sur le cycle.

Le nombre maximum de substituants susceptibles d'être portés par un cycle, est aisément déterminé par l'Homme du Métier.

Dans le cas d'un carbocycle saturé, le nombre maximum de substituants est égal à $2\rho - 3$, $\rho$ représentant le nombre d'atomes de carbone du cycle. Ce nombre est diminué de 2, chaque fois qu'il y a une double liaison ou des cycles accolés.

Généralement, le nombre de substituants présents sur un cycle varie de 1 à 5 et est le plus souvent égal à 1, 2 ou 3.

En ce qui concerne la nature des substituants, des exemples de substituants sont donnés ci-après mais cette liste ne présente pas de caractère limitatif.

N'importe quel substituant peut être présent sur le cycle dans la mesure où il n'interfère pas au niveau du produit désiré. On donne ci-après des exemples de substituants qui peuvent être portés par le reste A :

- R peut représenter $R_0$, l'un des groupes suivants :

  . un radical aliphatique acyclique, linéaire ou ramifié, ayant de 1 à 20 atomes de carbone, saturé ou comprenant une ou plusieurs insaturations sur la chaîne, de préférence 1 à 3 insaturations qui sont de préférence, des doubles liaisons simples ou conjuguées ; la chaîne hydrocarbonée pouvant être éventuellement :

. interrompue par l'un des groupes suivants dénommés Z :
-O- ; -CO- ; COO- ; $-NR_2-$ ; $-CO-NR_2-$ ; -S- ; $-SO_2-$ ;
dans lesdites formules $R_2$ représente un atome d'hydrogène, un radical alkyle linéaire ou ramifié ayant de 1 à 6 atomes de carbone,

. et/ou porteuse de l'un des substituants suivants :
-OH ; -CN ; $-N[R_2]_2$ ; $-COOR_2$ ; $-CF_3$ ou -X ;
dans lesdites formules, les radicaux $R_2$, identiques ou différents, représentent un atome d'hydrogène, un radical alkyle linéaire ou ramifié ayant de 1 à 6 atomes de carbone et X représente. un atome d'halogène, de préférence, de fluor, de chlore, de brome,

. un radical du type $=R_3$, dans lequel $R_3$ représente un radical alkylidène ayant de 1 à 6 atomes de carbone, un radical de formule $=C(CN)_2$ ou un radical cycloalkylidène ou cycloalcénylidène ayant 5 ou 6 atomes de carbone ou un radical benzylidène éventuellement substitué, de préférence par un atome d'halogène X,

. un radical alkoxy linéaire ou ramifié ayant de 1 à 6 atomes de carbone,

. deux atomes successifs du cycle peuvent être reliés entre eux par un pont époxy ou par un pont du type alkylène dioxy ayant de 1 à 4 atomes de carbone, de préférence les radicaux méthylène dioxy, éthylène dioxy, propylène dioxy,

. un groupe OH,

. un groupe $COOR_4$, $R_4$ représentant un atome d'hydrogène ou un radical alkyle ayant de 1 à 4 atomes de carbone, de préférence méthyle, éthyle,

. un groupe CN,

. un atome d'halogène, de préférence, de fluor, de chlore, de brome.

. un groupe-$CF_3$.

- R peut représenter $R_1$, l'un des groupes plus complexes suivants :

. un radical carbocyclique saturé ou insaturé ayant de 4 à 7 atomes de carbone, de préférence un radical cyclopentyle, cyclohexyle, cyclopentène-2 yle, cyclopentène-3 yle, cyclohexène-1 yle, cyclohexène-2 yle, cyclohexène-3 yle,

. un radical de formule

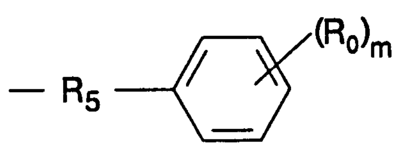

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

. un radical - $R_5$ - Z - $R_8$ dans lequel Z et $R_5$ ont la signification donnée précédemment, $R_8$ représente un radical alkyle linéaire ou ramifié ayant de 1 à 6 atomes de carbone ou un radical de formule

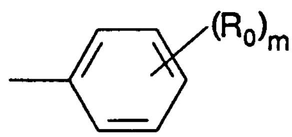

dans lequel $R_0$ et m ont la signification donnée précédemment,

. un radical de type spiro de formule :

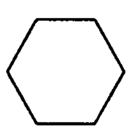 ou 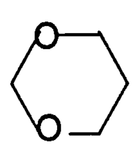 ou 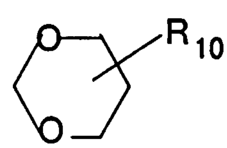

dans laquelle $R_{10}$ représentant un ou plusieurs radicaux alkyle, linéaires ou ramifiés ayant de 1 à 6 atomes de carbone.

Plus précisément, dans la formule (Ia), les divers symboles précédents peuvent prendre les significations données ci-après.

A peut représenter le reste d'un composé carbocyclique monocyclique saturé, ayant de 3 à 20 atomes de carbone. Il peut y avoir présence d'un ou de deux groupes carbonyle sur le cycle. Le groupe carbonyle est de préférence porté par un carbocycle saturé ayant de 5 ou 6 atomes de carbone.

Le carbocycle saturé peut porter des substituants. Le nombre de substituants sur chaque cycle, peut varier largement de 1 à 5. Il est généralement de 1 ou 2.

Comme exemples spécifiques de substituants on peut mentionner :

. un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,
. un radical alkyle ayant de 1 à 15 atomes de carbone porteur d'un groupe fonctionnel, de préférence une fonction OH, CN, $N[R_2]_2$, $COOR_2$ avec $R_2$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2.CH_2-CO-(CH_2)_4-CH_3$,
. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule $-C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,
. un radical $=C(CH_3)_2$ ou $=CH-(CH_2)_2-CH_3$,
. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_2$ dans laquelle $R_2$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule$-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de fommule $-CH=CH-CO-CH_3$, un radical de formule $-CH=CH-CO-(CH_2)_4-CH_3$ ou un radical de formule $-CH=CH-CHOH-(CH_2)_4-CH_3$,
. un radical alkoxy linéaire ou ramifié ayant de 1 à 6 atomes de carbone, de préférence un radical méthoxy, éthoxy,
. deux atomes successifs du cycle peuvent être reliés entre eux par un pont époxy ou par un pont tel que les radicaux méthylène dioxy, éthylène dioxy, propylène dioxy,
. un radical de type spiro de formule

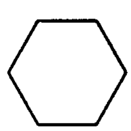 ou 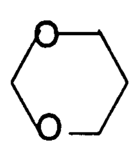 ou 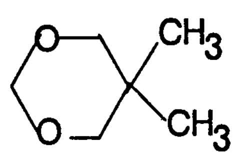

. un radical de formule

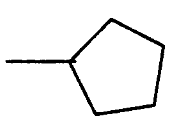 ou 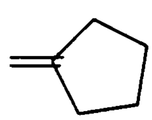 ou 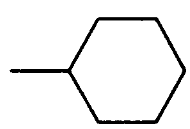 ou 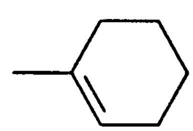

. un radical de formule

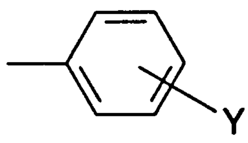 ou 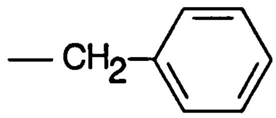 ou 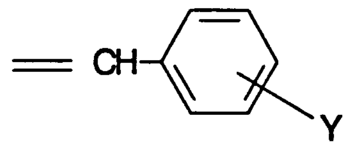

Y représentant un atome d'hydrogène, un atome d'halogène, de préférence de fluor ou de chlore,

. un radical de formule

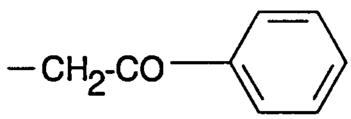 ou 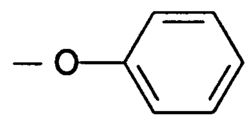

. un groupe OH,
. un groupe $COOR_4$, $R_4$ représentant un atome d'hydrogène ou un radical alkyle ayant de 1 à 4 atomes de carbone, de préférence méthyle, éthyle,
. un groupe CN,
. un atome d'halogène, de préférence, de fluor, de chlore, de brome.

A titre illustratif, on peut citer plus particulièrement les composés de formule suivante :

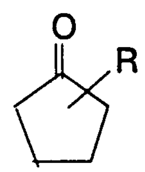 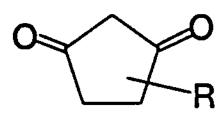 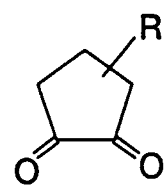

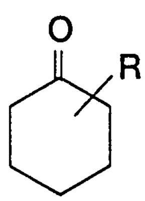 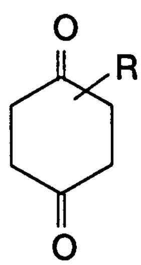 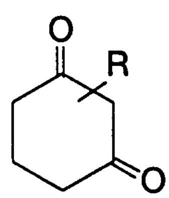 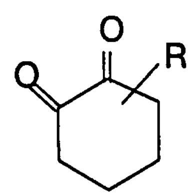

A peut représenter le reste d'un composé carbocyclique monocyclique insaturé, ayant de 4 à 20 atomes de carbone. Il peut y avoir présence d'un ou de deux groupes carbonyle sur le cycle. Le groupe carbonyle est de préférence porté par un carbocycle insaturé ayant de 5 ou 6 atomes de carbone.

Le carbocycle insaturé peut porter des substituants. Le nombre de substituants sur chaque cycle, peut varier largement de 1 à 5. Il est généralement de 1 ou 2.

Comme exemples spécifiques de substituants on peut mentionner :

. un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,
. un radical alkyle ayant de 1 à 15 atomes de carbone porteur d'un groupe fonctionnel, de préférence une fonction OH, CN, $N[R_2]_2$, $COOR_2$ avec $R_2$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de fomule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2.CH_2-CO-(CH_2)_4-CH_3$,

. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule _$C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,

. un radical $=C(CH_3)_2$ ou $=CH-(CH_2)_2-CH_3$,

. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_2$ dans laquelle $R_2$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,

. un radical alkoxy linéaire ou ramifié ayant de 1 à 6 atomes de carbone, de préférence un radical méthoxy, éthoxy,

. un radical de formule

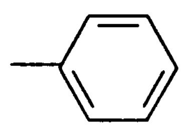

. un groupe OH,

. un groupe COOR, R représentant un atome d'hydrogène ou un radical alkyle ayant de 1 à 4 atomes de carbone,de préférence méthyle, éthyle,

. un atome d'halogène, de préférence, de fluor, de chlore, de brome.

A titre illustratif, on peut citer plus particulièrement les composés de formule suivante :

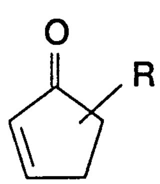 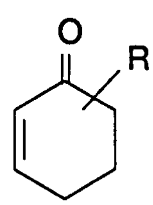 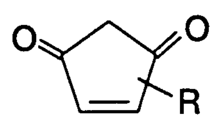 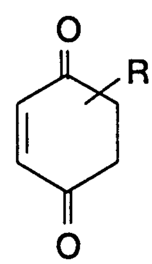

A peut représenter le reste d'un composé carbocyclique polycyclique, de préférence, bicyclique comprenant deux carbocycles saturés, ayant chacun de préférence de 4 à 8 atomes de carbone. Il peut y avoir présence d'un groupe carbonyle sur l'un ou les deux cycles. Il est également possible qu'il y ait deux groupes carbonyle sur le même cycle. Le groupe carbonyle est de préférence porté par un ou deux carbocycles saturés ayant de 5 ou 6 atomes de carbone.

Dans ces composés polycycliques, un ou plusieurs atomes de carbone, de préférence deux, peuvent être remplacés par un hétéroatome, de préférence un atome d'azote ou d'oxygène.

Le ou les cycles de ce composé polycyclique peuvent porter des substituants. Le nombre de substituants sur chaque cycle est généralement de 1 à 4, de préférence 1 ou 2. Comme exemples de substituants couramment rencontrés, on peut citer :

. un radical alkyle linéaire ou ramifié ayant de 1 à 6 atomes de carbone, de préférence un radical méthyle, isopropyle,

. un radical de formule $-CH_2Br$,

. un radical alkoxy linéaire ou ramifié ayant de 1 à 6 atomes de carbone, de préférence un radical méthoxy,

- un radical de formule $=CH_2$,
- un groupe OH,
- un groupe -COOH,
- un atome d'halogène, de préférence, de fluor, de chlore, de brome,
- un groupe $CF_3$

A titre illustratif, on peut citer plus particulièrement les composés de formule suivante :

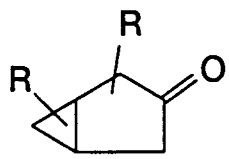
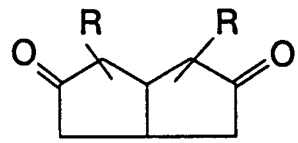
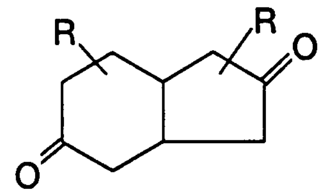
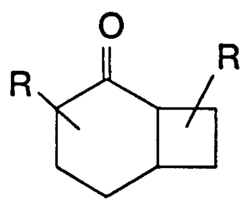
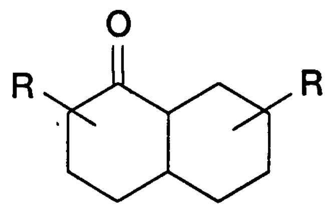
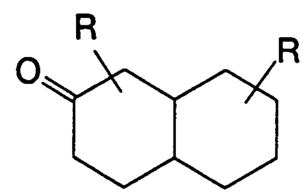
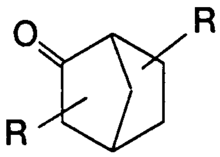
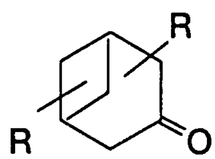
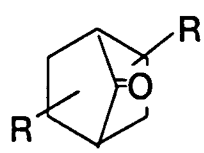
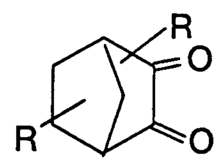
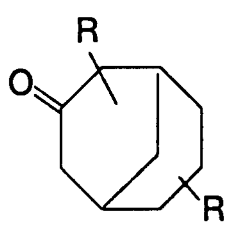
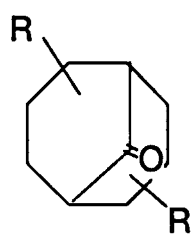
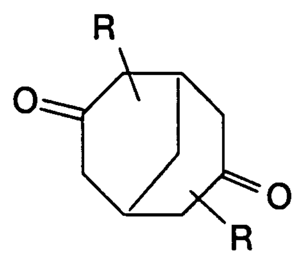
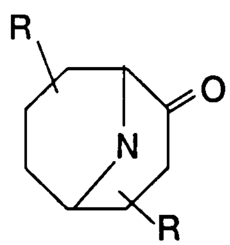
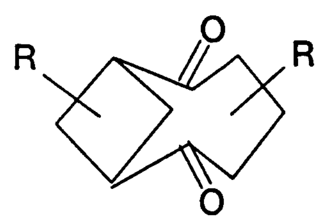

A peut représenter le reste d'un composé carbocyclique bicyclique comprenant deux carbocycles, ayant chacun de préférence de 4 à 7 atomes de carbone, l'un saturé, l'autre insaturé, généralement avec une seule double liaison. Le groupe carbonyle peut intervenir aussi bien dans le cycle saturé qu'insaturé ou sur les deux. Le groupe carbonyle est de préférence porté par un carbocycle saturé ou insaturé, ayant de 5 ou 6 atomes de carbone.

Le ou les cycles de ce composé polycyclique peuvent porter des substituants. Le nombre de substituants sur chaque cycle est généralement de 1 à 3, de préférence 1 ou 2.

Comme exemples spécifiques de substituants on peut mentionner :

- un radical alkyle linéaire ou ramifié ayant de 1 à 6 atomes de carbone, de préférence un radical méthyle,
- un radical de formule

$$-\underset{\displaystyle CH_3}{\underset{|}{C}}=CH_2,$$

. un radical de formule $-CH_2-O-CH_3$,
. un atome d'halogène, de préférence, de chlore.

A titre illustratif, on peut citer plus particulièrement les composés de formule suivante :

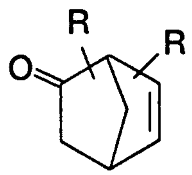
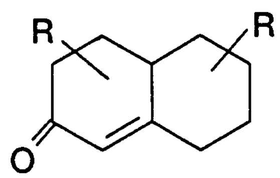
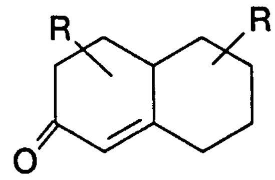

A peut représenter le reste d'un composé carbocyclique polycyclique, de préférence, bicyclique comprenant deux carbocycles insaturés, ayant chacun de préférence de 5 ou 6 atomes de carbone. Il peut y avoir présence d'un groupe carbonyle sur l'un des deux cycles.

Dans ces composés polycycliques, un ou plusieurs atomes de carbone, de préférence deux, peuvent être remplacés par un hétéroatome, de préférence un atome d'azote ou d'oxygène.

Le ou les cycles de ce composé polycyclique peuvent porter des substituants. Le nombre de substituants sur chaque cycle est généralement de 1 à 5, de préférence 1 ou 2.

Généralement, les substituants sont un ou plusieurs radicaux alkyle linéaire ou ramifié ayant de 1 à 6 atomes de carbone, de préférence un radical méthyle, éthyle.

A titre illustratif, on peut citer plus particulièrement les composés de formule suivante :

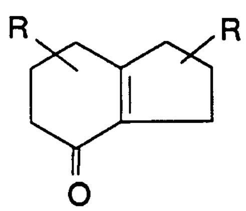
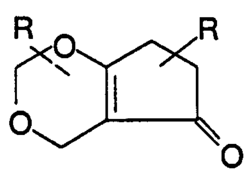
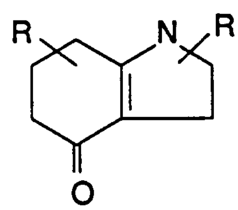
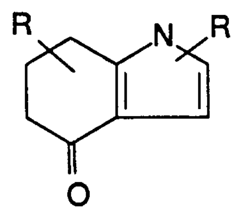

A peut représenter le reste d'un composé carbocyclique polycyclique comprenant au moins un carbocycle aromatique, de préférence, un cycle benzénique et un carbocycle ayant de préférence de 4 à 7 atomes de carbone et comprenant un ou deux groupes carbonyle.

A est de préférence le reste d'un composé bicyclique comprenant un cycle benzénique et un carbocycle de 5 ou 6 atomes de carbone comprenant un ou deux groupes carbonyle.

Les deux cycles de ce radical bicyclique peuvent porter des substituants. Le nombre de substituants sur chaque cycle est généralement de 1 ou 2.

Comme exemples spécifiques de substituants on peut mentionner :

. un radical alkyle linéaire ou ramifié ayant de 1 à 6 atomes de carbone, de préférence un radical méthyle, tert-butyle,
. un radical de formule

=C-CN,
|
CN

. un radical alkoxy linéaire ou ramifié ayant de 1 à 6 atomes de carbone, de préférence un radical méthoxy,
. un radical alkoxy linéaire ou ramifié ayant de 1 à 6 atomes de carbone porteur d'autres groupes fonctionnels tels que, par exemple, un groupe OH et/ou $N[R_2]_2$ avec $R_2$, identiques ou différents, représentant un atome d'hydrogène, un radical alkyle linéaire ou ramifié ayant de 1 à 6 atomes de carbone, de préférence un radical de formule $-O-CH_2-CHOH-CH_2-NHBu-t$
. un groupe OH,
. un groupe acyle ayant de 2 à 6 atomes de carbone, de préférence un radical acétyle ou de formule -CO-tert-butyle,
. un groupe $-CH_2-COOH$,
. un groupe $-NH_2$,
. un atome d'halogène, de préférence, de fluor, de chlore, de brome.

A titre illustratif, on peut citer plus particulièrement les composés de formule suivante :

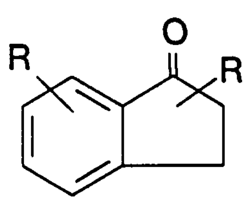
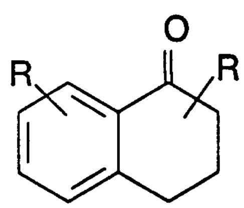
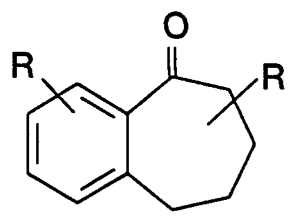

Parmi tous les composés cétoniques cycliques précités, le procédé de l'invention s'applique plus particulièrement, aux composés cétoniques carbocycliques ayant au moins un atome d'hydrogène ou une fonction ester en position $\alpha$ par rapport au groupe carbonyle, saturés ou insaturés, ayant de 5 à 6 atomes de carbone dans le cycle et répondant à la formule (Ia) dans laquelle :

- R représente un atome d'hydrogène, un radical alkyle ou alkoxy linéaire ou ramifié, ayant de 1 à 6 atomes de carbone, de préférence, de 1 à 4 atomes de carbone, un radical benzylidène éventuellement porteur d'un atome d'halogène,
- n est égal à 1.

Les cétones cycliques répondant à la formule (Ia) mises en oeuvre préférentiellement dans le procédé de l'invention sont choisies parmi :

- celles dans lesquelles le reste A de la formule (Ia) représente le reste d'un composé monocyclique saturé comprenant un seul groupe carbonyle, telles que :
  - cyclobutanone,
  - cyclopentanone,
  - 2-méthylcyclopentanone,
  - 3-méthylcyclopentanone,
  - 2-méthyl-2-carboxyméthylcyclopentanone
  - 2,2-diméthylcyclopentanone,
  - 2-(2-octényl)-cyclopentanone,
  - 2-(3,7-diméthyl-2,6-octadiényl) cyclopentanone,
  - 2-cyclopentylidènecyclopentanone,
  - 2-benzylidènecyclopentanone,
  - 2-[(p-chloro)benzylidène]cyclopentanone,
  - 2-méthyl-2-carboxyméthyl-5-[(p-chloro)benzylidène]cyclopentanone

- 2,4-diméthylcyclopentanone,
- 2,5-diméthylcyclopentanone,
- 3,4-diméthylcyclopentanone,
- 2,2,4-triméthylcyclopentanone,
- 5-méthyl-2-(1-méthyléthylidène)-cyclohexanone,
- 6-cétoprostaglandine E1,
- méthylester prostaglandine E2,
- prostaglandine D2,
- cyclohexanone,
- 3-méthylcyclohexanone,
- 4-n-pentylcyclohexanone,
- 2-benzylidènecyclohexanone,
- 2-(N,N,-diméthylamino)cyclohexanone,
- 3,5-diméthylcyclohexanone,
- dihydrocarvone,
- cycloheptanone,
- cyclooctanone,
- cycloheptadécanone,

- celles dans lesquelles le reste A de la formule (Ia) représente le reste d'un composé monocyclique saturé comprenant deux groupes carbonyle, telles que :

  - 1,3-cyclopentanedione
  - 2-allyl-2-méthyl-1,3-cyclopentanedione,
  - 3,3-diméthyl-1,2-cyclopentanedione,
  - 3,4-diméthyl-1,2-cyclopentanedione,
  - 1,2-cyclohexanedione,
  - 1,3-cyclohexanedione,
  - 1,4-cyclohexanedione,
  - 1,2-cycloheptanedione,

- celles dans lesquelles le reste A de la formule (Ia) représente le reste d'un composé monocyclique insaturé comprenant un seul groupe carbonyle, telles que :

  - 2-cyclopenténone,
  - 3-méthyl-2-cyclopenténone,
  - 4,4-diméthyl-2-cyclopenténone,
  - 2-pentyl-2-cyclopenténone,
  - 3-éthoxy-2-cyclopenténone,
  - 2-hydroxy-3-éthyl-2-cyclopenténone,
  - prostaglandine J2,
  - jasmone,
  - 2-hydroxy-3,4-diméthyl-2-cyclopenténone,
  - 15-oxoprostaglandine E2,
  - 2-éthoxy-2-cyclohexénone,
  - 3-bromo-2-cyclohexénone,
  - carvone,
  - 8-hydroxycarvotanacétone,
  - 2-méthyl-5-(1-méthyléthènyl)-2-cyclohexénone,
  - 3,5,5-triméthyl-2-cyclohexénone,
  - méthyl ester de l'acide abscisique,
  - 2-hydroxy-3-méthyl-6-(1-méthyléthyl)-2-cyclohexénone,
  - 5-cyclohexadécènone.

- celles dans lesquelles le reste A de la formule (Ia) représente le reste d'un composé monocyclique insaturé comprenant deux groupes carbonyle, telles que :

  - 2-cyclopentène-1,4-dione,
  - 4-hydroxy-5-méthyl-4-cyclopentène-1,3-dione,

- celles dans lesquelles le reste A de la formule (Ia) représente le reste d'un composé bicyclique saturé comprenant un ou deux groupes carbonyle, telles que :

  - camphor,
  - norcamphor,
  - 3-bromocamphor,
  - 2,3-bornanedione,
  - 1-décalone,
  - 2-décalone,
  - N-(éthoxycarbonyl)nortropinone,

- celles dans lesquelles le reste A de la formule (Ia) représente le reste d'un composé bicyclique saturé/insaturé comprenant un ou deux groupes carbonyle, telles que :

  - bicyclo[3.2.0]hept-2-en-6-one,
  - 1-(méthoxyméthyl)-bicyclo[2.2.0]hept-5-en-2-one,
  - 3,4,8,8a-tétrahydro-8a-méthyl-1,6(2H,7H)-naphtalènedione.

- celles dans lesquelles le reste A de la formule (Ia) représente le reste d'un composé bicyclique insaturé comprenant un groupe carbonyle, telles que :

  - 6,7-dihydro-cyclopenta-1,3-dioxin-5(4H)-one
  - 6,7-dihydro-1,1,2,3,3-pentaméthyl-4(5H)-indanone,
  - 4-oxo-4,5,6,7-tétrahydroindole.

- celles dans lesquelles le reste A de la formule (Ia) représente le reste d'un composé bicyclique dont l'un d'eux est aromatique comprenant un ou deux groupes carbonyle, telles que :

  - 2-indanone,
  - 2-méthyl-1-indanone,
  - 4-méthyl-1-indanone,
  - 4-méthoxy-1-indanone,
  - 6-méthoxy-1-indanone,
  - 4-hydroxy-1-indanone,
  - 5-bromo-1-indanone,
  - 1,3-indanedione,
  - 1-tétralone,
  - 2-tétralone,
  - 4-méthyl-1-tétralone,
  - 5,7-diméthyl-1-tétralone,
  - 5-méthoxy-1-tétralone,
  - 6,7-diméthoxy-1-tétralone,
  - 5-hydroxy-1-tétralone,
  - levobunolol.

Lorsque le substrat de départ représente un composé cétonique acyclique, il peut être symbolisé par la formule chimique suivante :

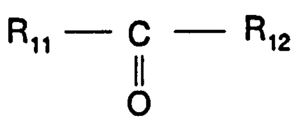

(Ib)

dans ladite formule (Ib) :

- $R_{11}$ et $R_{12}$, identiques ou différents représentent un radical hydrocarboné ayant de 1 à 20 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique ; un radical aliphatique saturé ou insaturé, linéaire ou ramifié, porteur d'un substituant cyclique.

- au moins l'un des radicaux $R_{11}$ et $R_{12}$ comprend un atome de carbone en position $\alpha$ par rapport au groupe carbonyle qui porte au moins un atome d'hydrogène ou une fonction ester.

Dans la formule générale (Ib) des composés cétoniques, $R_{11}$ et $R_{12}$, identiques ou différents peuvent représenter un radical aliphatique acyclique, saturé ou insaturé, linéaire ou ramifié.

Plus précisément, $R_{11}$ et $R_{12}$ représentent un radical aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ; la chaîne hydrocarbonée pouvant être éventuellement interrompue par l'un des groupes tels que par exemple, les groupes précités Z ou et/ou porteuses de l'un des substituants suivants, précédemment définis : -OH ; -CN ; $-N[R_2]_{2;}$;$-COOR_2$ ; $-CF_3$ ou -X.

Le reste aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le reste aliphatique acyclique peut être relié au cycle par un lien valentiel ou par l'un des groupes tels que dénommés Z.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un substituant quelconque.

Dans la formule générale (Ib) des composés cétoniques acycliques, l'un des radicaux $R_{11}$ ou $R_{12}$ peut également représenter un radical hydrocarboné cyclique, saturé ou non ayant de préférence 5 ou 6 atomes de carbone dans le cycle ; un radical hétérocyclique, saturé ou non, comportant notamment 5 ou 6 atomes dans le cycle dont 1 ou 2 hétéroatomes tels que les atomes d'azote, de soufre et d'oxygène; un radical hydrocarboné aromatique, monocyclique ou polycyclique condensé ou non.

Comme exemples de composés cétoniques mis en oeuvre dans le procédé de l'invention, on peut mentionner entre autres :

- cétones aliphatiques saturées
  - acétone,
  - méthyléthylcétone,
  - méthylisopropylcétone,
  - méthylisobutylcétone,
  - 2-pentanone,
  - 3-pentanone,
  - 2-carboxyméthyl-3-pentanone
  - 2-hexanone,
  - 3-hexanone,
  - 5-méthyl 2-hexanone,
  - 2-heptanone,
  - 3-heptanone,
  - 4-heptanone,
  - 2-octanone,
  - 3-octanone,
  - diisobutylcétone,
  - 5-méthyl 2-octanone
  - 2-nonanone,
  - 2,6,8-triméthyl 4-nonanone
- cétones porteuses d'un groupe fonctionnel
  - 1,3-dihydroxy 2-propanone,
  - diacétonealcool,
  - triacétonedialcool,
  - 4-méthoxy 4-méthyl 2-pentanone,
- cétones aliphatiques insaturées
  - oxyde de mésityle,
  - 3-butène 2-one,

- 4-méthyl 4-pentène 2-one,

- dicétones aliphatiques

  - 2,3-pentanedione,
  - 2,3-hexanedione,
  - 3,4-hexanedione,
  - 4-méthyl 2,3-pentanedione,
  - 3,4-heptanedione,
  - 5-méthyl 2,3-hexanedione,
  - 2,3-octanedione,
  - 4,5-octanedione,
  - 2,5-diméthyl 3,4-hexanedione,
  - 5-méthyl 3,4-heptanedione,
  - 6-méthyl 3,4-heptanedione,
  - 1-phényl 1,2-propanedione,
  - 2,4-pentanedione,
  - 2,4-hexanedione,
  - 2,4-heptanedione,
  - 1-phényl 1,3-butanedione,
  - 1-phényl 1,3-pentanedione,
  - 1,3-diphényl 1,3-propanedione,
  - 1-phényl 2,4-pentanedione,
  - 2,5-hexanedione,
  - 3,4-diméthyl 2,5-hexanedione,
  - 3,3,4,4-tétraméthyl 2,5 hexanedione,
  - 2,5-heptanedione,
  - 3,6-octanedione,
  - 6-méthyl 2,5-heptanedione,
  - 2,5-décanedione,
  - 2,5-dodécanedione,
  - 1,4-diphényl 1,4-butanedione,

- cétones mixtes aliphatiques et carbo- ou hétérocycliques

  - acétophénone,
  - propiophénone,
  - 2,2-diéthoxyacétophénone,
  - acétylpyrazine,
  - 2-acétylpyridine,
  - 3-acétylpyridine,
  - 4-acétylpyridine,
  - 2-acétylpyrrole,
  - 2-acétyl 1-tétralone.

Dans le procédé de l'invention, on conduit la réaction de C-alkylation du composé cétonique à l'aide d'un agent d'alkylation, en présence d'un catalyseur.

Plusieurs types d'agents d'alkylation conviennent pour la mise en oeuvre du procédé de l'invention. Ainsi, on peut faire appel à un ester organique et plus particulièrement aux esters organiques répondant à la formule générale (II) :

$$[R_a - CO - O -]_p - R_b \qquad (II)$$

dans laquelle :

- $R_a$ représente :

  . un radical $R_c$ qui peut être un radical hydrocarboné ayant de 1 à 12 atomes de carbone qui peut être un radical aliphatique saturé ou insaturé, linéaire ou ramifié, éventuellement porteur d'un atome d'halogène, de préférence, un atome de chlore ; un radical carbocyclique saturé, insaturé ou aromatique, monocyclique ou polycyclique ; un radical aliphatique acyclique, saturé ou insaturé, linéaire ou ramifié porteur d'un substituant cyclique,

. un radical $R_d$ - O - CO - $R_f$ - dans lequel le radical $R_d$ a la signification donnée pour le radical $R_c$ et le radical $R_f$ représente un lien valentiel ou un radical divalent aliphatique, saturé ou insaturé, linéaire ou ramifié ayant au moins 1 atome de carbone,

. un radical $R_d$ - O - dans lequel le radical $R_d$ a la signification donnée pour le radical $R_c$,

. un radical $R_d$ - O - CO - O - dans lequel le radical $R_d$ a la signification donnée pour le radical $R_c$,

- $R_b$ représente :

  . un radical $R_c$ tel que précédemment défini,

  . un métal alcalin ou alcalino-terreux,

- $R_a$ et $R_b$ peuvent former ensemble un radical divalent aliphatique saturé ou insaturé, linéaire ou ramifié, ayant au moins 2 atomes de carbone.
- p est un nombre entier égal à 1 ou 2.

La formule générale (II) recouvre aussi bien des esters d'acides carboxyliques que des carbonates organiques ou des carbonates mixtes organométalliques.

Parmi les composés répondant à la formule générale (II), on met en oeuvre plus particulièrement les esters d'acides carboxyliques de formule (IIa) :

$$R_a - CO - O - R_b \qquad (IIa)$$

dans laquelle :

- $R_a$ représente :

  . un radical alkyle linéaire ou ramifié ayant de 1 à 6 atomes de carbone, de préférence, de 1 à 3 atomes de carbone,

  . un radical cycloalkyle ayant 5 ou 6 atomes de carbone, de préférence, 6 atomes de carbone,

  . un radical aralkyle ayant de 7 à 12 atomes de carbone,

  . un radical $R_c$ - O - CO - $R_d$ - dans lequel le radical $R_c$ a la signification donnée ci-dessus pour le radical $R_a$ et le radical divalent $R_d$ représente un lien valentiel ou un radical alkylène ayant de 1 à 6 atomes de carbone.

- $R_b$ représente :

  . un radical alkyle, linéaire ou ramifié ayant de 1 à 6 atomes de carbone,

  . un radical cycloalkyle ayant 5 ou 6 atomes de carbone,

- $R_a$ et $R_b$ peuvent former ensemble un radical alkylène ayant de 2 à 4 atomes de carbone.

Les esters organiques mis en oeuvre préférentiellement, répondent à la formule (IIa) dans laquelle $R_a$ représente un radical alkyle, linéaire ou ramifié, ayant de 1 à 6 atomes de carbone, de préférence, de 1 à 4 atomes de carbone et $R_b$, un radical alkyle, linéaire ou ramifié, ayant de 1 à à 4 atomes de carbone.

Comme exemples d'esters d'acides carboxyliques répondant à la formule (IIa), on peut citer les esters d'alkyle, de préférence méthyle ou éthyle des acides monocarboxyliques aliphatiques saturés tels que l'acide acétique, propionique, butyrique, isobutyrique, valérique, isovalérique, pivalique, et des acides dicarboxyliques aliphatiques saturés tels que l'acide oxalique, l'acide succinique, glutarique, adipique.

Parmi les différents esters précités, on choisit de mettre en oeuvre préférentiellement pour des raisons économiques l'acétate de méthyle ou l'acétate d'éthyle.

Conviennent également à la mise en oeuvre du procédé de l'invention, les carbonates organiques et les carbonates mixtes organo-métalliques.

Les carbonates organiques et les carbonates mixtes organo-métalliques utilisés dans l'invention sont plus particulièrement les carbonates de formule générale (IIb) :

$$[R_a - O - CO - O -]_p - R_b \qquad (IIb)$$

dans laquelle:

- $R_a$ représente :

  . un radical alkyle, linéaire ou ramifié, ayant 1 à 6 atomes de carbone et éventuellement porteur d'un atome d'halogène, de préférence, un atome de chlore,
  . un radical cycloalkyle ayant 5 à 6 atomes de carbone,
  . un radical aryle ayant de 6 à 12 atomes de carbone,
  . un radical $R_d$ - O - CO - dans lequel $R_d$ représente un radical alkyle, linéaire ou ramifié ayant 1 à 6 atomes de carbone, un radical cycloalkyle ayant 5 ou 6 atomes de carbone ;

- $R_b$ représente :

  . un radical alkyle, linéaire ou ramifié, ayant 1 à 6 atomes de carbone,
  . un radical cycloalkyle ayant 5 ou 6 atomes de carbone,
  . un métal alcalin ou alcalino-terreux, de préférence le sodium ou le potassium,
  p = 1 ou p = 2 lorsque $R_b$ représente un métal alcalino-terreux ;

- $R_a$ et $R_b$ peuvent former ensemble un radical alkylène ayant 2 à 6 atomes de carbone.

Les carbonates organiques mis en oeuvre préférentiellement, répondent à la formule (IIb) dans laquelle $R_a$ et $R_b$, identiques ou différents, représentent un radical alkyle, linéaire ou ramifié, ayant de 1 à 6 atomes de carbone, de préférence, de 1 à 4 atomes de carbone.

Comme exemples de carbonates organiques ou organo-métalliques, on peut citer : le carbonate de ditertiobutyle, le carbonate de diéthyle, le carbonate de diméthyle, le carbonate d'éthylène, le carbonate de propylène, le carbonate de phényle et de tertiobutyle, le carbonate de tertiobutyle et de sodium, le dicarbonate de ditertiobutyle, le chlorométhylméthylcarbonate.

Comme autres agents d'alkylation, on peut avoir recours aux dialkylsulfates et plus particulièrement aux dialkylsulfates de formule $R_g$-O-$SO_2$-O-$R_h$, dans ladite formule, $R_g$ et $R_h$, identiques ou différents représentent un radical alkyle, linéaire ou ramifié, ayant de 1 à 6 atomes de carbone.

On fait appel préférentiellement, au diméthylsulfate.

On peut également utiliser, comme agent d'alkylation, un alcool de préférence, un alcool répondant à la formule (IIc) :

$$R_i - OH \qquad \text{(IIc)}$$

dans ladite formule, $R_i$ représente un radical hydrocarboné ayant de 1 à 12 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

Parmi les alcools répondant à la formule (IIc), on choisit préférentiellement les alcools inférieurs et préférentiellement ceux répondant à la formule (IIc) dans laquelle $R_i$ représente un radical alkyle linéaire ou ramifié ayant de 1 à 6 atomes, de préférence, de 1 à 4 atomes de carbone.

On choisit tout particulièrement, le méthanol ou l'éthanol.

Conformément au procédé de l'invention, on effectue la réaction de C-alkylation d'un composé cétonique, en présence d'un catalyseur comprenant un anion orthophosphate condensé ou non.

Selon le procédé de l'invention, on peut engager l'orthophosphate, sous n'importe quelle forme. Toutefois, pour des raisons économiques, il est préférable d'avoir recours aux formes commerciales facilement accessibles.

Il existe trois séries d'orthophosphates : les dihydrogénophosphates, les monohydrogénophosphates et les phosphates.

On fait appel de préférence aux orthophosphates mais il est également possible de mettre en oeuvre des phosphates condensés c'est-à-dire des phosphates contenant plus d'un atome de phosphore. Ils sont formés d'un enchaînement de motifs tétraédriques $PO_4$ unis entre eux par des atomes d'oxygène. Ces motifs peuvent former par exemple des chaînes de polyphosphates linéaires contenant de 2 à 10 atomes de phosphore. Comme exemples spécifiques, on peut citer les anions respectivement avec 2 ou 3 atomes de phosphore tels que pyrophosphate $P_4O_7{}^{2-}$ ou tripolyphosphate $P_3O_{10}{}^{5-}$.

En ce qui concerne le contre-anion, il peut être de nature quelconque. Il peut s'agir d'un élément métallique et plus particulièrement un élément du groupe 1a, 2a ou 3b de la Classification périodique des éléments ou bien un cation ammonium.

Pour la définition des éléments, on se réfère ci-après à la Classification périodique des éléments publiée dans le Bulletin de la Société Chimique de France, n°1 (1966).

Le catalyseur mis en oeuvre est de préférence, un orthophosphate métallique ou d'ammonium. Il convient également de faire appel à leurs mélanges ou à leurs sels mixtes.

Comme exemples spécifiques de phosphates convenant tout à fait bien à l'invention, on peut mentionner :

- l'orthophosphate de sodium,
- l'orthophosphate de potassium,
- l'orthophosphate d'aluminium,
- l'orthophosphate d'ammonium,
- l'orthophosphate d'argent,
- l'orthophosphate de baryum,
- l'orthophosphate de calcium,
- l'orthophosphate de chrome,
- l'orthophosphate de cobalt,
- l'orthophosphate de cuivre,
- l'orthophosphate double de magnésium et d'ammonium,
- l'orthophosphate de fer,
- l'orthophosphate de lithium,
- l'orthophosphate de magnésium,
- l'orthophosphate de manganèse,
- l'orthophosphate de potassium,
- l'orthophosphate de zinc,
- le monohydrogénophosphate de sodium,
- le monohydrogénophosphate de calcium,
- le monohydrogénophosphate de magnésium,
- le monohydrogénophosphate de zirconium,
- le pyrophosphate de sodium,
- le pyrophosphate de potassium,
- le pyrophosphate de calcium,
- le pyrophosphate de cuivre
- le pyrophosphate de zinc,
- le pentapolyphosphate de sodium $Na_7P_5O_{16}$,
- le tripolyphosphate de sodium $Na_5P_3O_{10}$,
- le tripolyphosphate de potassium $K_5P_3O_{10}$.

Le phosphate intervenant dans le procédé de l'invention peut être mis en oeuvre sous forme anhydre ou hydratée.

Parmi tous les catalyseurs cités, les catalyseurs choisis préférentiellement sont les orthophosphates, les pyrophosphates, les tripolyphosphates ou les pentapolyphosphate de sodium ou de potassium.

Une variante préférée de l'invention consiste à faire appel à un orthophosphate d'un métal du groupe 3a à savoir un orthophosphate d'une terre rare trivalente que l'on peut symboliser par la formule (III) :

$$MPO_4 \qquad \text{(III)}$$

dans laquelle :

- M représente une terre rare trivalente à savoir un lanthanide ayant un numéro atomique de 57 à 71 et l'yttrium ainsi que le scandium.

Parmi les catalyseurs susceptibles d'être mis en oeuvre dans le procédé de l'invention, on peut faire appel à une première famille qui regroupe les orthophosphates de terres rares légères également dénommées terres rares cériques incluant les éléments La, Ce, Pr, Nd, Sm, Eu. Lesdits orthophosphates sont dimorphiques. Ils présentent une structure hexagonale et évoluent vers une structure monoclinique lorsqu'ils sont chauffés vers une température de 600 - 800°C.

Une deuxième famille d'orthophosphates de terres rares convenant à l'invention regroupent les orthophosphates de Gd, Tb et Dy. Ceux-ci présentent la même structure que les orthophosphates de terres rares cériques mais présentent en plus, une troisième phase cristalline de structure quadratique à haute température (vers 1700°C).

Une troisième famille d'orthophosphates de terres rares regroupent les orthophosphates de terres rares lourdes appelées également terres rares yttriques incluant Y, Ho, Er, Tm, Yb et Lu. Lesdits composés cristallisent uniquement sous la forme quadratique.

Parmi les différentes classes d'orthophosphates de terres rares précitées, on fait appel préférentiellement aux orthophosphates de terres rares légères.

Les catalyseurs préférés de l'invention répondent à la formule (III) dans laquelle M est le lanthane, le cérium et le samarium.

On ne sortira pas du cadre de la présente invention à faire appel à tout composé oxygéné du phosphore qui, lors de la synthèse du catalyseur ou lors de la réaction, conduit à un orthophosphate d'un métal de terre rare.

Les orthophosphates des métaux de terres rares de départ, mis en oeuvre dans le procédé de l'invention sont des produits connus. On peut faire appel aux phosphates du commerce, notamment à l'orthophosphate de lanthane ou bien les synthétiser selon les procédés décrits dans la littérature.

Si l'on se réfère aux techniques générales de fabrication de phosphates [notamment au "PASCAL P. Nouveau traité de chimie minérale, tome X (1956), pp.821-823" et "Gmelins Handbuch der anorganischen Chemie (8ième édition) vol.16 (C), pp. 202-206 (1965)"], on peut distinguer deux voies principales d'accès aux phosphates : d'une part, la précipitation d'un sel soluble du métal (chlorure, nitrate) par l'hydrogénophosphate d'ammonium ou l'acide phophorique : d'autre part, la réaction de l'oxyde de métal avec de l'acide phosphorique à chaud. Dans les deux cas, on peut envisager un traitement de finition avec un hydroxyde alcalin.

Les orthophosphates desdits métaux peuvent être également préparés par chamottage (réaction solide-solide) de leurs sels avec des sels de phosphore, puis calcination.

On peut se référer, plus particulièrement, pour la préparation :

- de l'orthophosphate de cérium, aux travaux de FUKUO et al [Nippon Kagakkai Shi (Revue de l'Association japonaise de Chimie (4), pp.622-626 (1975)],
- de l'orthophosphate de lanthane, à l'article J.M. COWLEY et al [Journal of Catalysis, 56, pp.185-194 (1979)],
- de l'orthophosphate d'yttrium à la publication de L.S. ESHCHENKO et al [Russian Journal of Inorganic Chemistry, 30, (6), (1985)].

Le produit est ensuite séché selon les techniques classiques connues de l'Homme du Métier. Il se fait avantageusement entre 50°C et 200°C, pendant une durée allant de préférence, de 2 à 8 heures, sous atmosphère normale ou sous pression réduite (par exemple 10 mm de mercure = 1300 Pa) ou par lyophilisation.

Le produit séché peut être ensuite calciné à une température comprise entre 200°C et 1000°C, de préférence entre 400°C et 800°C, pendant une durée variant de 1 à 15 heures, de préférence de 3 à 6 heures.

Une autre variante du procédé de l'invention consiste à faire appel à un orthophosphate de terre rare trivalente dopé par un métal alcalin ou un métal alcalino-terreux.

Dans l'exposé qui suit de la présente invention, on appelle "agent dopant" l'élément alcalin et/ou alcalino-terreux.

Plus précisément, le catalyseur de l'invention répond à la formule suivante :

$$MPO_4\ (Im)_p \qquad \text{(IIIa)}$$

dans laquelle :

- M représente $M_3$ une terre rare trivalente ou un mélange d'au moins une terre trivalente $M_3$ et d'au moins un élément choisi parmi les métaux alcalins $M_1$ et les métaux alcalino-terreux $M_2$ avec la relation :

$$M = \alpha M_1^{\ +} + \beta M_2^{\ ++} + \gamma M_3^{\ 3+} \text{ et } \alpha + 2\beta + 3\gamma = 3$$

- Im correspond à un composé d'imprégnation basique constitué de métal alcalino-terreux, de préférence de métal alcalin et leurs mélanges associés à un contre-anion pour assurer la neutralité électrique.
- $\alpha$ est un coefficient compris entre 0 et 3, avantageusement supérieur à 0,01 et au plus égal à 0,5, de préférence compris entre 0,05 et 0,2.
- $\beta$ est un coefficient compris entre 0 et 3/2, de préférence entre 0 et 1/3 ou bien 1 $\pm$ 0,1.
- $\gamma$ est un coefficient compris entre 0 et 1, avantageusement au moins égal à 1/3, de préférence à 1/2,
- p est inférieur à 0,5 et avantageusement compris entre 0,04 et 0,25.

Dans la formule (IIIa), les différents éléments sont les suivants :

- $M_1$ est choisi dans le groupe des éléments de la colonne 1a et leurs mélanges, de préférence les métaux alcalins tels que le lithium, le sodium, le potassium, le rubidium, le césium et le francium,
- $M_2$ est choisi dans le groupe des éléments de la colonne 2a et leurs mélanges, de préférence les métaux alcalino-terreux tels que le béryllium, le magnésium, le calcium, le strontium, le baryum, le radium,
- $M_3$ est choisi dans le groupe des terres rares trivalentes choisies parmi les lanthanides, l'yttrium le scandium et leurs mélanges, de préférence les lanthanides tels que le lanthane, le cérium, le praséodyme, le néodyme, le

samarium, l'europium, le gadolinium, le terbium, le dysprosium, l'holmium, l'erbium, le thulium, l'ytterbium et le lutécium.

En général, M est constitué d'au plus trois éléments et ce pour des raisons de commodités ; pour les mêmes raisons, il peut être économiquement intéressant d'utiliser des mélanges commerciaux de terres rares sous quelle que forme qu'ils soient dès lors qu'ils conduisent aisément au composé selon l'invention ; ainsi bien que cela ne soit guère critique, il est fréquent que $\gamma$ soit peu différent de un (O,9 $\pm$ 0,1) et que M ne comporte qu'un métal aux impuretés près.

Im correspond à un composé d'imprégnation constitué d'un métal choisi parmi les alcalins et alcalino-terreux, de préférence, les métaux alcalins, et leurs mélanges associés à un ou plusieurs contre-anions pour assurer la neutralité éléctrique.

Im est avantageusement différent de $MPO_4$, notamment lorsque l'agent d'imprégnation est un alcalino-terreux.

Le(s) contre-anion(s) initial(aux), c'est-à-dire avant traitement thermique sont choisis de préférence parmi les anions nitrate, sulfate, chlorure, fluorure, hydrogénophosphate, phosphate, hydrogénosulfate, sulfate, etc...

Les anions peuvent être d'une seule espèce ou être constitués d'un mélange des espèces ; pour des raisons de simplicité on préfère n'avoir qu'une seule espèce ou qu'une seule famille d'espèces.

La teneur du catalyseur en agent dopant est généralement telle que le pourcentage pondéral de l'agent dopant par rapport au phosphate de terre trivalente soit compris entre 0 % et 25 %, de préférence entre 3 et 15%.

Les catalyseurs mis en oeuvre dans le procédé de l'invention sont des produits connus car décrits dans la demande de brevet EP-A 0 440 555. Pour leur préparation, on pourra se référer à la demande de brevet EP-A 0 440 555 qui est incorporée par référence à la présente demande.

Un mode de fabrication desdits composés consiste à réaliser l'imprégnation d'un composé de formule $MPO_4$, M ayant été défini antérieurement avec une solution d'imprégnant Imp tel que défini, dans un solvant volatil, de préférence l'eau.

Le produit $MPO_4$ peut être chimiquement modifié par imprégnation à sec ou voie humide.

Ainsi, un mode de préparation consiste à effectuer l'imprégnation à sec de l'orthophosphate métallique $MPO_4$, à l'aide d'une solution d'au moins un sel de métal alcalin ou alcalino-terreux. Comme mentionné précédemment, les contre-anions préférés sont l'hydrogénophosphate ou le phosphate, de préférence l'hydrogénophosphate de césium.

L'imprégnation est réalisée à sec c'est-à-dire que le volume total de solution utilisée est approximativement égal au volume poreux total présenté par l'orthophosphate du métal de terre rare trivalente. On sèche et calcine le produit obtenu.

Plus précisément, l'imprégnation à sec consiste à ajouter à une masse $m_1$ d'une poudre du produit à imprégner un volume V d'une solution aqueuse d'un ou des sels de cations ou d'anions à fixer sur la surface du solide. Le volume V de solution est choisie tel que $V/m_1$ soit égal au volume poreux à l'eau du solide à imprégner.

La concentration C en cations ou anions de la solution imprégnante est choisie telle que le rapport $CVM_2/m_1$ soit égal au pourcentage en poids choisi d'espèce imprégnante fixée sur la surface du produit à imprégner (avec $M_2$ = masse molaire de l'espèce imprégnante). On procède goutte à goutte à l'ajout de la solution de manière à obtenir une adsorption homogène.

Le produit peut ensuite être laissé au repos pendant un temps variable à température ambiante. Le produit est ensuite séché suivant les techniques classiques connues de l'Homme du Métier. Le séchage est généralement conduit à la pression atmosphérique ou sous pression réduite ou lyophilisation. Il peut être également calciné.

L'imprégnation voie humide se fait en dispersant l'orthophosphate d'un métal de terre rare trivalente, dans une solution aqueuse de sels de cations et/ou d'anions à fixer sur la surface du solide.

Cette solution peut présenter une concentration variant de $10^{-3}$ M à 10 M en l'espèce imprégnante.

Le pH de la solution pourra être avantageusement ajusté à une valeur au moins égale à celle du point isoélectrique du produit à modifier pour fixer préférentiellement les cations (cas usuel) ; toutefois, cette condition n'est pas impérative. Il est possible, au-dessous de ce point isoélectrique, de fixer correctement les cations lorsque les anions associés ont un caractère très "covalent" comme les sulfates et les phosphates.

La température de la solution peut varier de l'ambiante à 100°C.

La dispersion est agitée vigoureusement, pendant un temps variable.

Le produit est ensuite filtré et éventuellement lavé.

Dans ces deux variantes de préparation desdits composés chimiques, on précisera que le séchage est conduit avantageusement à une température variant de 50°C à 200°C, pendant une durée allant de préférence, de 2 à 8 heures.

En ce qui concerne l'opération de calcination, celle-ci est conduite à une température comprise entre 200°C et 1000°C, de préférence entre 400°C et 800°C, pendant une durée variant de 1 à 15 heures, de préférence de 3 à 6 heures.

Les catalyseurs préférés de l'invention répondent à la formule (III) dans laquelle M est le lanthane, le cérium et le samarium éventuellement dopé par un métal alcalin, de préférence le césium.

Les catalyseurs selon la présente invention sont tels que la surface des corps catalytiques soit formée pour partie

au moins d'un composé selon la présente invention.

La phase catalytique peut être utilisée pure ou être supportée. Dans la suite de la description, on désignera par corps catalytique, le catalyseur dans sa forme particulaire unitaire, que le catalyseur soit supporté ou non. Le dépôt de la phase catalytique sur le support est faite selon des techniques connues de l'Homme du métier.

Ces corps catalytiques peuvent être de toute forme en elles-mêmes connues pour des catalyseurs solides utilisables en phase gazeuse.

Le reste dudit corps catalytique, c'est-à-dire pour l'essentiel la partie n'entrant pas en contact avec le mélange gazeux réactionnel peut être en matériau(x) quelconque(s), pourvu qu'il(s) soi(en)t inerte(s) dans les conditions d'usage ; pour des raisons de facilité de fabrication, il peut être réalisé en composés choisis dans le groupe constitué par les phosphates, les mono- et dihydrogénophosphates et leur mélange. Les catalyseurs peuvent être également réalisés entièrement en composés chimiques (III) ou (IIIa) selon la présente invention.

Le catalyseur peut se présenter sous différentes formes : poudre, ou produits mis en forme tels que granulés (par exemple cylindres), billes, pastilles ou monolithes (blocs en forme de nid d'abeilles) qui sont obtenus par extrusion, moulage, compactage ou tout autre type de procédé connu.

La surface spécifique du catalyseur est la plus élevée possible, en général au moins égal à 1 $m^2$, avantageusement au moins égal à 10 $m^2$, le plus souvent entre 50 et 150 $m^2/g$, de préférence entre 50 et 100 $m^2/g$.

Le choix du catalyseur intervenant dans le procédé de l'invention dépend de la nature du substrat de départ.

Lorsque le composé cétonique de départ est mis à réagir avec un agent d'alkylation c'est-à-dire qu'il ne présente pas de fonction ester en position $\alpha$ par rapport au groupe carbonyle, le catalyseur est choisi essentiellement comme étant un orthophosphate de terre rare trivalente répondant à la formule (III).

Dans le cas où le composé cétonique présente une fonction ester en position $\alpha$ par rapport au groupe carbonyle, la gamme de catalyseurs convenant tout à fait bien est élargie et l'on peut faire appel à tout orthophosphate, dihydrogénophosphate, monohydrogénophosphate ou phosphate condensé et plus particulièrement aux orthophosphates de terres rares trivalentes de formule (III) ainsi qu'aux orthophosphates de terres rares dopés de formule (IIIa).

Conformément au procédé de l'invention, la réaction de C-alkylation est conduite en phase vapeur ou en phase liquide, en mettant en contact le composé cétonique de départ avec l'agent d'alkylation (si nécessaire), en présence du catalyseur tel que défini.

La quantité d'agent d'alkylation utilisée est égale ou supérieure à la quantité stoechiométrique nécessaire pour substituer un ou plusieurs atomes d'hydrogène. Par plusieurs atomes d'hydrogène, on entend au plus quatre atomes d'hydrogène par groupe carbonyle.

Généralement, l'agent d'alkylation est mis en oeuvre en une quantité telle que le rapport entre le nombre de moles de l'agent d'alkylation et le nombre d'atomes d'hydrogène remplacés par un groupe alkyle d'un composé cétonique varie entre 0,5 et 500, de préférence entre 1 et 10.

En ce qui concerne le catalyseur, sa productivité pondérale et horaire est comprise entre 0,1 et 20 $h^{-1}$, de préférence entre 1 et 5 $h^{-1}$: la productivité pondérale horaire d'un catalyseur étant définie par le rapport pondéral entre le composé cétonique introduit par heure et le catalyseur.

D'un point de vue pratique, le procédé de l'invention peut être conduit en phase vapeur ou en phase liquide.

Un premier mode d'exécution de l'invention, qui est préféré, consiste à conduire le procédé de l'invention en phase vapeur. On entend par cette expression que les différents réactifs sont vaporisés dans les conditions réactionnelles mais le procédé n'exclut pas la présence d'une éventuelle phase liquide résultant soit des propriétés physiques des réactifs, soit d'une mise en oeuvre sous pression ou de l'utilisation d'un solvant organique.

Le gaz vecteur est optionnel et est en général un gaz ou un mélange de gaz non réactifs dans les conditions de la réaction. On peut faire appel à des gaz tel que l'azote, l'air, l'argon ou l'hélium. Avantageusement, le rapport volumique entre le gaz vecteur et le composé cétonique varie entre 0 et 10, de préférence entre 0,1 et 2,0.

La température de la réaction de C-alkylation est généralement comprise entre 80°C et 500°C, et, de préférence de 200°C et 350°C.

La pression réactionnelle peut être comprise entre $10^{-2}$ et 50 bars, de préférence la pression atmosphérique.

Conformément au procédé de l'invention, on vaporise les réactifs de départ à savoir le composé cétonique et l'agent d'alkylation. Ils sont introduits au contact du catalyseur, de préférence entraînés par un gaz vecteur.

Le temps de contact qui est défini comme le rapport entre le volume apparent de catalyseur et le débit du flux gazeux (qui inclut le gaz vecteur), peut varier largement, et est le plus souvent compris entre 0,2 et 100 secondes. Le temps de contact est choisi de préférence entre 0,4 et 10 secondes.

En ce qui concerne la réalisation pratique de l'invention, on commence à préparer le lit catalytique qui est constitué par la phase active catalytique déposée sur un support (par exemple, verre fritté) ce qui permet la circulation des gaz sans élution du catalyseur. Ensuite, les réactifs sont mis en oeuvre et plusieurs variantes sont possibles.

Il est possible de vaporiser chacun des réactifs, composé cétonique, agent d'alkylation, dans des chambres différentes, puis d'effectuer le mélange dans une chambre de mélange et d'introduire le flux gazeux résultant sur le catalyseur. Le gaz vecteur peut être introduit en parallèle audit flux gazeux ou bien au niveau de la chambre de mélange.

Une autre variante consiste à préparer une solution comprenant le composé cétonique, l'agent d'alkylation, puis à

vaporiser ledit mélange et à l'introduire sur le catalyseur, en parallèle avec le gaz vecteur.

Un autre mode de réalisation pratique de l'invention consiste à faire fondre le composé cétonique en la chauffant jusqu'à sa température de fusion et l'on fait passer au-dessus un flux gazeux comprenant l'agent d'alkylation et l'eau éventuellement formée au cours de la réaction. Ce flux se sature en composé cétonique et il est mis alors en contact avec le catalyseur.

Un autre mode d'exécution de l'invention est de faire appel à un solvant organique, inerte dans les conditions réactionnelles et qui est choisi de telle sorte qu'il solubilise le composé cétonique et l'agent d'alkylation mis en oeuvre.

On fait appel de préférence selon l'invention à un solvant aprotique ayant un point d'ébullition élevé supérieur à 80°C et, de préférence, compris entre 80°C et 300°C.

A titre de solvants aprotiques susceptibles d'être mis en oeuvre dans le procédé de l'invention, on peut citer les hydrocarbures aliphatiques ou aromatiques comme l'hexane, l'heptane, le cyclohexane, le benzène, le toluène, les xylènes ; les hydrocarbures chlorés comme le dichlorobenzène ; les éthers cycliques comme le tétrahydrofurane, le dioxanne ; les sulfones comme le diméthylsulfoxyde, le sulfolane ; les carboxamides cycliques comme la N-méthylpyrrolidone ; les nitriles aromatiques comme le benzonitrile.

On peut également utiliser plusieurs solvants.

La quantité de composé cétonique mise en oeuvre dans le solvant est généralement telle que le rapport molaire solvant/composé cétonique soit compris entre 0 et 20 et, de préférence, entre 0 et 5.

Ainsi, on prépare une solution organique comprenant le composé cétonique, l'agent d'alkylation, puis l'on vaporise ledit mélange et on l'introduit sur le catalyseur, en parallèle avec le gaz vecteur.

En fin de réaction, on condense l'ensemble des gaz et l'on sépare les réactifs non réagis et les produits obtenus, par distillation ou cristallisation fractionnée. Il est également possible de séparer ceux-ci, par condensation fractionnée.

Un autre mode de réalisation pratique de l'invention est de travailler en phase liquide, éventuellement en présence d'un solvant organique.

Les réactifs, et en particulier l'agent d'alkylation peuvent être utilisés en tant que solvant réactionnel mais il est possible de faire appel à un solvant organique.

Plusieurs impératifs président au choix du solvant organique.

Il doit être stable dans les conditions réactionnelles et inerte vis-à-vis des réactifs et produits obtenus

Il doit présenter une température d'ébullition élevée, de préférence comprise entre 200°C et 500°C.

Comme exemples de solvants convenant particulièrement à l'invention, on peut mentionner entre autres :

- les hydrocarbures aliphatiques et/ou aromatiques et plus particulièrement les paraffines tels que notamment, le décane, le undécane, le dodécane ou le tétradécane ; les hydrocarbures aromatiques comme notamment les xylènes, le cumène, les coupes pétrolières constituées de mélange d'alkylbenzènes notamment les coupes de type Solvesso®,
- les éthers et plus particulièrement les éthers aromatiques comme l'oxyde de biphényle et/ou l'oxyde de benzyle,
- les huiles paraffiniques et/ou naphténiques, résidus de distillation du pétrole.

On peut également mettre en oeuvre un mélange de solvants organiques.

Interviennent donc dans le procédé de l'invention, le composé cétonique de départ, l'agent d'alkylation si nécessaire, le catalyseur de la réaction mis en oeuvre dans les quantités précédemment mentionnées et le solvant organique.

La concentration du composé cétonique dans la masse réactionnelle peut varier largement et représente généralement de 10 à 50 % du poids de la masse réactionnelle.

D'un point de vue pratique, on charge dans un autoclave, tous les réactifs dans un ordre quelconque et l'on chauffe.

La température de la réaction de C-alkylation est généralement comprise entre 80°C et 500°C, et, de préférence de 200°C et 350°C.

La pression réactionnelle peut être comprise entre 1 et 50 bars, de préférence entre 1 et 10 bars.

En fin de réaction, on récupère le composé cétonique selon les techniques classiques utilisées dans ce domaine technique, notamment par distillation, décantation ou par cristallisation.

Le procédé de l'invention permet d'obtenir préférentiellement une cétone cyclique ayant de 1 à 2 groupes alkyle R' en position $\alpha$ par rapport au groupe carbonyle et répondant à la formule (IV) suivante :

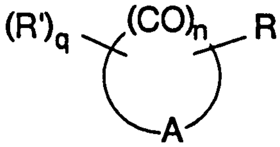

(IV)

dans laquelle :

- A symbolise le reste d'un cycle cyclopentane ou cyclohexane,
- R représente un atome d'hydrogène, un radical alkyle ou alkoxy, linéaire ou ramifié, ayant de 1 à 6 atomes de carbone, de préférence, de 1 à 4 atomes de carbone, un radical benzylidène éventuellement porteur d'un atome d'halogène,
- R' représente un radical alkyle, linéaire ou ramifié, ayant de 1 à 6 atomes de carbone, de préférence, de 1 à 4 atomes de carbone,.
- n est égal à 1,
- q est un nombre compris entre 1 et 4, de préférence égal à 1 ou 2.

Le procédé de l'invention est parfaitement bien adapté à la préparation de la 2-méthylcyclopentanone à partir de la cyclopentanone ou de la 2-méthyl-2-carboxyméthylcyclopentanone ; de la 2,2-diméthylcyclopentanone à partir de la cyclopentanone et/ou de la 2-méthylcyclopentanone ou de la 2-méthyl-2-carboxyméthylcyclopentanone.

Il convient également pour la préparation de la 2,2-diméthyl-5-[(p-chloro)benzylidène]cyclopentanone, à partir de la 2-[(p-chloro)benzylidène]cyclopentanone ou de la 2-méthyl-2-carboxyméthyl-5-[(p-chloro)benzylidène]cyclopentanone.

Les exemples qui suivent illustrent l'invention sans toutefois la limiter.

## EXEMPLES

Dans les différents exemples qui suivent, les abréviations TT, RR, RT ont la signification suivante :

$$\text{Taux de transformation} = TT = \frac{\text{nombre de moles de cétone transformée}}{\text{nombre de moles de cétone introduite}} \text{ en \%}$$

$$\text{Rendement Réel} = RR = \frac{\text{nombre de moles de cétone C-alkylée formée}}{\text{nombre de moles de cétone introduite}} \text{ en \%}$$

$$\text{Sélectivité} = RT = \frac{\text{nombre de moles de cétone C-alkylée formée}}{\text{nombre de moles de cétone transformée}} \text{ en \%}$$

### Exemple 1

On introduit dans un réacteur, 106 g de $H_3PO_4$ (à 85% vendu par la société Prolabo) soit 0,92 mole dans 800 ml d'eau désionisée.

On agite à 500-700 tours /minute.

On introduit 299 g de $La_2(CO_3)_3$, $12H_2O$ à froid, (soit 0,8 mole de lanthane dans 736 ml d'eau), lentement et sous forte agitation.

On chauffe ensuite le milieu réactionnel jusqu'à 80°C, pendant deux heures trente.

On laisse refroidir jusqu'à température ambiante sans agitation pendant une demi-heure et on finit dans un bain d'eau froide.

La suspension est filtrée sur un verre fritté n°3 jusqu'à épuisement des eaux-mères.

Le produit est alors redispersé dans 2 litres d'eau, sous forte agitation et laissé en suspension pendant une demi-heure, tout en maintenant l'agitation.

La suspension est filtrée sur un verre fritté n°3 jusqu'à épuisement des eaux-mères

Le produit est remis en dispersion dans 900 ml d'eau désionisée et neutralisé par une solution d'ammoniaque jusqu'à un pH égal à 9.

Le produit est filtré, lavé à l'eau et centrifugé avant d'être séché à 110°C.

Le produit est ensuite calciné à 700°C pendant 3 heures.

### Exemple 2

Dans un réacteur tubulaire en pyrex d'un volume de 20 ml, on charge successivement 5 ml de quartz, 0.5 ml du catalyseur phosphate de lanthane préparé selon l'exemple 1 et encore 5 ml de quartz.

On introduit en tête de réacteur, en parallèle, de l'azote à l'aide d'un débimètre et un mélange de 2,01 g de 2-méthylcyclopentanone (MCP), en solution dans le diméthylcarbonate (DMC) à l'aide d'un pousse-seringue : le rapport molaire diméthylcarbonate/2-méthylcyclopentanone est de 3.

A la sortie du réacteur, les effluents gazeux sont condensés dans un piège refroidi par un bain eau-glace. Les piégats sont ensuite dilués puis analysés par chromatographie en phase gazeuse .

La réaction est conduite en utilisant un débit d'azote de 2 l/h et un débit d'alimentation de la solution de 1,98 ml/h.

Après 1 h de réaction à 250°C, on observe les performances suivantes :

- $TT_{2\text{-méthylcyclopentanone}}$ = 32.6 %
- $RT_{2,2\text{-diméthylcyclopentanone}}$ = 36.8 %
- $RT_{2,2,5\text{-triméthylcyclopentanone}}$ (TMCP)= 16 %

Exemple 3

On introduit dans un réacteur, 182,4 g de $H_3PO_4$ (à 85% vendu par la société Prolabo) soit 1,6 mole dans 640 ml d'eau désionisée.

On chauffe le milieu réactionnel jusqu'à 85°C et quand la température est atteinte, on introduit 260,8 g de $La_2O_3$ (soit 0,8 mole de lanthane) de façon régulière (14 g toutes les 5 minutes).

On laisse chauffer le mélange réactionnel 1 heure à 85°C, puis on laisse refroidir jusqu'à température ambiante sous agitation.

Le produit est récupéré par centrifugation (3600 tours/mn), puis redispersé dans 400 ml d'eau désionisée.

Le produit ainsi lavé est récupéré par centrifugation.

Cette opération de lavage est effectuée 3 fois suivant la même procédure.

Le produit ainsi lavé est séché à 110°C.

Le produit est ensuite calciné à 700°C pendant 3 heures.

Exemples 4 à 10

La 2,2-diméthylcyclopentanone est préparée comme dans l'exemple 2, avec le catalyseur décrit dans l'exemple 3.

Les perfomances obtenues sont consignées dans le tableau I.

Tableau I

| Ref. ex. | Température (°C) | Volume catalyseur (ml) | Quantité DMC+MCP (g) | Rapport molaire DMC/MCP | TT (%) | RT DMCP (%) | RT TMCP (%) |
|---|---|---|---|---|---|---|---|
| 4 | 350 | 0.5* | 2,06 | 3 | 44.9 | 42.7 | 23,3 |
| 5 | 250 | 0.5 | 2,41 | 3 | 47.2 | 43.4 | 23 |
| 6 | 200 | 0.5 | 2,30 | 3 | 13.6 | 31.6 | trace |
| 7 | 250** | 0.5 | 2,20 | 10 | 81.3 | 28 | 30 |
| 8 | 300 | 0.5 | 2,70 | 3 | 60.6 | 30.4 | 20 |
| 9 | 250 | 1.4 | 2,09 | 3 | 70.0 | 30.6 | 18,5 |
| 10 | 350 | 0.5 | 2,12 | 3 | 71.4 | 27.6 | 14,7 |

* = catalyseur préparé comme dans l'exemple 3 mais calciné à 550°C, pendant une nuit,

** présence de 2,2,5,5-tétraméthylcyclopentanone: RT = 25 %.

Exemples 11 à 16

La 2,2-diméthylcyclopentanone est préparée dans les conditions de l'exemple 2. Avec différents catalyseurs, on obtient les performances suivantes :

Tableau II

| Ref. ex. | Température (°C) | Catalyseur (0.5 ml) | Quantité DMC+MCP (g) | Rapport molaire DMC/MCP | TT (%) | RT DMCP (%) | RT TMCP (%) |
|---|---|---|---|---|---|---|---|
| 11 | 250 | SmPO4 | 2,03 | 3 | 33 | 57,1 | 15 |
| 12 | 250 | NdPO4 | 2,26 | 3 | 42,6 | 46,7 | 15 |
| 13 | 250 | YPO4 | 2,36 | 3 | 32 | 21,8 | 8,4 |
| 14 | 350 | YPO4 | 2,20 | 3 | 82,8 | 9 | 9,2 |
| 15 | 250 | ErPO4 | 2,20 | 3 | 34 | 29,7 | 13,5 |
| 16 | 250 | YbPO4 | 2,18 | 3 | 30,6 | 31,4 | 13,4 |

Exemple 17

La 2,2-diméthylcyclopentanone est préparée dans les conditions de l'exemple 2, avec le catalyseur de l'exemple 3, à partir de cyclopentanone.

On introduit un mélange de 2,22 g de cyclopentanone, en solution dans le diméthylcarbonate (DMC) : le rapport molaire diméthylcarbonate/cyclopentanone est de 6.

Après 1 h à 250°C, les performances sont les suivantes :

- $TT_{cyclopentanone}$ = 55.8 %
- $RR_{2\text{-méthylcyclopentanone}}$ = 21.7 %
- $RR_{2,2\text{-diméthylcyclopentanone}}$ = 9.9 %
- $RR_{2,2,5\text{-triméthylcyclopentanone}}$ = 6,7 %
- $RT_{2\text{-méthylcyclopentanone}}$ = 38.9 %
- $RT_{2,2\text{-diméthylcyclopentanone}}$ = 17.7 %
- $RT_{2,2,5\text{-triméthylcyclopentanone}}$ = 12 %

Exemple 18

Dans un réacteur tubulaire en pyrex d'un volume de 20 ml, on charge successivement 5 ml de quartz, 0,5 ml du catalyseur phosphate de lanthane préparé selon l'exemple 1 et encore 5 ml de quartz.

On introduit en tête de réacteur, en parallèle, de l'azote à l'aide d'un débimètre et un mélange de 2,49 g de 2-(p-chlorobenzylidène)cyclopentanone (BCP), en solution dans le diméthylcarbonate (DMC) à l'aide d'un pousse-seringue : le rapport molaire DMC/BCP est de 20.

A la sortie du réacteur, les effluents gazeux sont condensés dans un piège refroidi par un bain eau-glace. Les piégats sont ensuite dilués puis analysés par chromatographie en phase gazeuse .

La réaction est conduite en utilisant un débit d'azote de 2 l/h et un débit d'alimentation de la solution de 1,98 ml/h.

Après 1 h de réaction à 350°C, on observe les perfomances suivantes :

- $TT_{BCP}$= 100 %
- $RR_{2,2\text{-diméthyl-5-(p-chlorobenzylidène)cyclopentanone}}$ = 40,2%
- $RT_{2,2\text{-diméthyl-5-(p-chlorobenzylidène)cyclopentanone}}$ = 40,2 %

Exemples 19 à 21

La 2,2-diméthyl-5-(p-chlorobenzylidène)cyclopentanone est préparée comme dans l'exemple 18, avec le catalyseur de l'exemple 3.

Tableau III

| Ref. ex. | Température (°C) | Temps de contact (s) | Quantité DMC+BCP (g) | Rapport molaire DMC/BCP | TT (%) | RR (%) | RT (%) |
|---|---|---|---|---|---|---|---|
| 19 | 250 | 0,8 | 1,79 | 20 | 66 | 35 | 53 |
| 20 | 250 | 0,4 | 2,69 | 20 | 38,1 | 12 | 31,5 |
| 21 | 250 | 1,2 | 2,14 | 20 | 91 | 17,6 | 19,4 |

Exemple 22

Dans un autoclave en inox d'un volume de 125 ml, on charge successivement sous argon, 4 g de BCP, 2 g du catalyseur phosphate de lanthane préparé selon l'exemple 1 et 17,44 g de diméthylcarbonate.

On ferme l'autoclave est l'on agite à 200°C pendant 4h. Le rapport molaire DMC/BCP est de 10.

En fin de réaction, le mélange réactionnel est dilué au dichlorométhane dans une fiole jaugée puis analysé par chromatographie en phase gazeuse.

On obtient ainsi les performances suivantes :

- $TT_{BCP}$= 64%
- $RR_{2,2\text{-diméthyl-5-(p-chlorobenzylidene)cyclopentanone}}$ = 15%
- $RT_{2,2\text{-diméthyl-5-(p-chlorobenzylidene)cyclopentanone}}$ = 23,4 %

Exemple 23

Dans un réacteur tubulaire en pyrex d'un volume de 20 ml, on charge successivement 5 ml de quartz, 0.5 ml du catalyseur phosphate de lanthane préparé selon l'exemple 1 et encore 5 ml de quartz.

On introduit en tête de réacteur, en parallèle, de l'azote à l'aide d'un débimètre et un mélange de 2,35 g de 2-méthyl-2-carboxyméthylcyclopentanone (MCMCP), en solution dans le diméthylcarbonate (DMC) à l'aide d'un pousse-seringue : le rapport molaire diméthylcarbonate/MCMCP est de 3.

A la sortie du réacteur, les effluents gazeux sont condensés dans un piège refroidi par un bain eau-glace. Les piégats sont ensuite dilués puis analysés par chromatographie en phase gazeuse.

La réaction est conduite en utilisant un débit d'azote de 2 l/h et un débit d'alimentation de la solution de 1,98 ml/h.

Après 1 h de réaction à 250°C, on observe les perfomances suivantes :

- $TT_{MCMCP}$ = 25,5 %
- $RT_{\text{2-méthylcyclopentanone}}$ = 47,4%
- $RT_{\text{2,2-diméthylcyclopentanone}}$ = 12,1 %

Exemple 24

Dans cet exemple, on prépare un catalyseur à base de phosphate de lanthane dopé par le césium.

On prélève 4,7 ml d'une solution 6 M de CsOH, à laquelle on ajoute 14,12 ml d'une solution 1M de $H_3PO_4$. On ajoute alors la quantité d'eau nécessaire pour compléter à 50 ml.

On prend 50 g de produit préparé dans l'exemple 1 que l'on place dans un bécher de 200 ml.

On introduit goutte à goutte 20 ml de la solution d'imprégnation en écrasant les agglomérats formés et en homogénéisant bien.

On laisse le produit reposer une heure. On le séche une nuit à 110°C et on le calcine 2 heures à 500°C.

- Teneur en césium sur sec = 3%

Exemples 25 à 39

La 2,2-diméthylcyclopentanone est préparée comme dans l'exemple 23, sans addition de diméthylcarbonate.

Avec différents catalyseurs et conditions opératoires on obtient les performances suivantes:

Tableau IV

| Ref ex. | Température (°C) | Catalyseur (0.5 ml) | Temps de contact (s) | Quantité MCMCP (g) | TT (%) | RT MCP (%) | RT DMCP (%) | RT TMCP (%) |
|---|---|---|---|---|---|---|---|---|
| 25 | 350 | LaPO4 | 0.8 | 2.35 | 88.8 | 30.6 | 22.5 | 12.7 |
| 26 | 350 | LaPO4 | 0.4 | 2.34 | 83.4 | 19.7 | 16.3 | 7 |
| 27 | 350 | LaPO4 | 1 | 2,35 | 88 | 38 | 28 | 14 |
| 28 | 300 | LaPO4 | 0.8 | 2.16 | 39 | 48 | 18 | - |
| 29 | 400 | LaPO4 | 0.8 | 2.17 | 100 | 29.3 | 20.1 | 12.2 |
| 30 | 250 | NdPO4 | 0.8 | 1.39 | 67 | 20.4 | 12.5 | 5.4 |
| 31 | 350 | YbPO4 | 0.8 | 1.16 | 93 | 31.8 | 9.8 | 10.3 |
| 32 | 350 | ErPO4 | 0.8 | 1.26 | 93.6 | 28.5 | 15.1 | 4.8 |
| 33 | 350 | YPO4 | 0.8 | 1.43 | 96 | 24.3 | 17 | 17.9 |
| 34 | 350 | AlPO4 | 0.8 | 1.1 | 63.3 | 30.9 | 15.8 | 7.1 |
| 35 | 350 | SmPO4 | 0.8 | 1.43 | 93.5 | 37.6 | 21.7 | 8.3 |
| 36 | 350 | CaHPO4 | 0.8 | 1.93 | 25.4 | 15.7 | 23.2 | - |
| 37* | 350 | LaPO4/Cs | 0.8 | 1.43 | 40.3 | 40.2 | 20.6 | 7.7 |
| 38 | 350 | BiPO4 | 0.8 | 1.7 | 8.7 | 5.7 | - | - |
| 39 | 350 | Zr(HPO4) | 0.8 | 1.4 | 14.2 | 35.9 | - | - |

* = catalyseur préparé selon l'exemple 24.

Exemple 40

Dans un réacteur tubulaire en pyrex d'un volume de 20 ml, on charge successivement 5 ml de quartz, 0.5 ml du catalyseur phosphate de lanthane préparé selon l'exemple 1 et encore 5 ml de quartz.

On introduit en tête de réacteur, en parallèle, de l'note à l'aide d'un débimètre et 2,0 g de 2-méthyl-2-carboxyméthyl-5-(p-chlorobenzylidène)cyclopentanone (MCMBCP), en solution dans 4 ml de toluène à l'aide d'un pousse-seringue.

A la sortie du réacteur, les effluents gazeux sont condensés dans un piège refroidi par un bain eau-glace. Les piégats sont ensuite dilués puis analysés par chromatographie en phase gazeuse.

La réaction est conduite en utilisant un débit d'note de 2 l/h et un débit d'alimentation de la solution de 1,98 ml/h.

Après 1 h de réaction à 350°C, on observe les perfomances suivantes :

- $TT_{MCMBCP}$ = 100 %
- $RR_{2,2\text{-diméthyl-5-(p-chlorobenzylidène)cyclopentanone}}$ = 47,0

Exemple 41

La 2,2-diméthyl-5-(p-chlorobenzylidène)cyclopentanone est préparée comme dans l'exemple 40 en utilisant le phosphate de samarium comme catalyseur. Dans ces conditions, on observe les performances suivantes :

- $TT_{MCMBCP}$ = 100 %
- $RR_{2,2\text{-diméthyl-5-(p-chlorobenzylidène)cyclopentanone}}$ = 23 %

Exemple 42

Dans un réacteur tubulaire en pyrex d'un volume de 20 ml, on charge successivement 5 ml de quartz, 0.5 ml du

catalyseur phosphate de lanthane préparé selon l'exemple 1 et encore 5 ml de quartz.

On introduit en tête de réacteur, en parallèle, de l'azote à l'aide d'un débimètre et un mélange de 1,56 g de 3-pentanone, en solution dans le diméthylcarbonate (DMC) à l'aide d'un pousse-seringue : le rapport molaire diméthylcarbonate/3-pentanone est de 3.

A la sortie du réacteur, les effluents gazeux sont condensés dans un piège refroidi par un bain eau-glace. Les piégats sont ensuite dilués puis analysés par chromatographie en phase gazeuse.

La réaction est conduite en utilisant un débit d'azote de 2 l/h et un débit d'alimentation de la solution de 1,98 ml/h.

Après 1 h de réaction à 250°C, on observe les perfomances suivantes :

- $TT_{3\text{-pentanone}}$ = 41 %
- $RT_{2\text{-méthyl-3-pentanone}}$ = 29 %

**Revendications**

1. Procédé de C-alkylation d'un composé cétonique ayant au moins un atome d'hydrogène ou une fonction ester en position α par rapport au groupe carbonyle caractérisé par le fait que l'on conduit la réaction d'alkylation en position α par rapport au groupe carbonyle, en présence d'une quantité efficace d'un catalyseur comprenant un anion orthophosphate condensé ou non.

2. Procédé selon la revendication 1 caractérisé par le fait que l'on fait réagir un composé cétonique présentant au moins un atome d'hydrogène en position α du groupe carbonyle avec un agent d'alkylation, en présence dudit catalyseur.

3. Procédé selon la revendication 1 caractérisé par le fait que l'on met en contact un composé cétonique présentant au moins une fonction ester en position α par rapport au groupe carbonyle avec ledit catalyseur : la fonction ester étant de préférence, un radical de type $-COOR_e$ dans lequel $R_e$ est un radical alkyle linéaire ou ramifié ayant de 1 à 6 atomes de carbone, et de préférence, de 1 à 4 atomes de carbone et plus préférentiellement un radical méthyle ou éthyle.

4. Procédé selon l'une des revendications 1 à 3 caractérisé par le fait que le composé cétonique répond à la formule générale (Ia) :

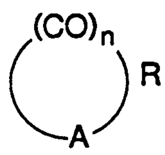

(Ia)

dans laquelle :

- A symbolise le reste d'un cycle formant tout ou partie d'un système monocyclique ou polycyclique comprenant au moins un groupe carbonyle et ayant au moins un atome d'hydrogène ou une fonction ester en position α par rapport au groupe carbonyle,
- R représente un atome d'hydrogène ou un ou plusieurs substituants, identiques ou différents,
- n est un nombre de préférence égal à 1 ou 2.

5. Procédé selon l'une des revendications 1 à 4 caractérisé par le fait que le composé cétonique répond à la formule (Ia) dans laquelle le reste A éventuellement substitué représente le reste :

- d'un composé monocyclique, carbocyclique, saturé ou insaturé,
- d'un composé polycyclique comprenant au moins deux carbocycles, saturés et/ou insaturés,
- d'un composé polycyclique comprenant au moins deux cycles saturés et/ou insaturés : un ou plusieurs des atomes de carbone pouvant être remplacés par un hétéroatome,
- d'un composé polycyclique comprenant au moins deux carbocycles dont l'un d'eux est aromatique.

6. Procédé selon l'une des revendications 1 à 5 caractérisé par le fait que le composé cétonique est un composé monocyclique carbocyclique avec un nombre d'atomes de carbone dans le cycle variant entre 3 et 20 atomes de

carbone, de préférence égal à 5 ou 6 atomes de carbone ; ledit carbocycle comprenant éventuellement 1 à 2 doubles liaisons.

7. Procédé selon l'une des revendications 1 à 5 caractérisé par le fait que le composé cétonique est un composé polycyclique avec un nombre d'atomes de carbone dans chaque cycle variant entre 3 et 8 atomes de carbone, de préférence égal à 5 ou 6 atomes de carbone.

8. Procédé selon l'une des revendications 1 à 5 caractérisé par le fait que le composé cétonique est un composé polycyclique comprenant au moins deux cycles saturés et/ou insaturés : un ou deux des atomes de carbone pouvant être remplacés par un hétéroatome, de préférence un atome d'oxygène ou d'azote.

9. Procédé selon l'une des revendications 1 à 8 caractérisé par le fait que le composé cétonique répond à la formule (Ia) dans laquelle le reste A porte un ou plusieurs substituants R tels que :

- R représentant $R_0$, l'un des groupes suivants :

  . un radical aliphatique acyclique, linéaire ou ramifié, ayant de 1 à 20 atomes de carbone, saturé ou comprenant une ou plusieurs insaturations sur la chaîne, de préférence 1 à 3 insaturations qui sont de préférence, des doubles liaisons simples ou conjuguées ; la chaîne hydrocarbonée pouvant être éventuellement :

    . interrompue par l'un des groupes suivants dénommés Z :
    -O- ; -CO- ; COO- ; $-NR_2-$ ; $-CO-NR_2-$ ; -S- ; $-SO_2-$ ;
    dans lesdites formules $R_2$ représente un atome d'hydrogène, un radical alkyle linéaire ou ramifié ayant de 1 à 6 atomes de carbone,
    . et/ou porteuse de l'un des substituants suivants :
    -OH ; -CN ; $-N[R_2]_2$ ; $-COOR_2$ ; $-CF_3$ ou -X ;
    dans lesdites formules, les radicaux $R_2$, identiques ou différents, représentent un atome d'hydrogène, un radical alkyle linéaire ou ramifié ayant de 1 à 6 atomes de carbone et X représente. un atome d'halogène, de préférence, de fluor, de chlore, de brome,

  . un radical du type $=R_3$, dans lequel $R_3$ représente un radical alkylidène ayant de 1 à 6 atomes de carbone, un radical de formule $=C(CN)_2$ ou un radical cycloalkylidène ou cycloalcénylidène ayant 5 ou 6 atomes de carbone ou un radical benzylidène éventuellement substitué, de préférence par un atome d'halogène X,
  . un radical alkoxy linéaire ou ramifié ayant de 1 à 6 atomes de carbone,
  . deux atomes successifs du cycle peuvent être reliés entre eux par un pont époxy ou par un pont du type alkylène dioxy ayant de 1 à 4 atomes de carbone, de préférence les radicaux méthylène dioxy, éthylène dioxy, propylène dioxy,
  . un groupe OH,
  . un groupe $COOR_4$, $R_4$ représentant un atome d'hydrogène ou un radical alkyle ayant de 1 à 4 atomes de carbone, de préférence méthyle, éthyle,
  . un groupe CN,
  . un atome d'halogène, de préférence, de fluor, de chlore, de brome.
  . un groupe $-CF_3$.

- R représentant $R_1$, l'un des groupes plus complexes suivants :

  . un radical carbocyclique saturé ou insaturé ayant de 4 à 7 atomes de carbone, de préférence un radical cyclopentyle, cyclohexyle, cyclopentène-2 yle, cyclopentène-3 yle, cyclohexène-1 yle, cyclohexène-2 yle, cyclohexène-3 yle,
  . un radical de formule

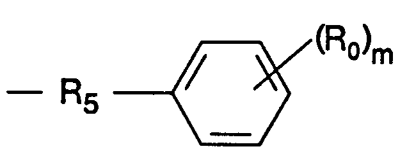

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

. un radical - $R_5$ - Z - $R_8$ dans lequel Z et $R_5$ ont la signification donnée précédemment, $R_8$ représente un radical alkyle linéaire ou ramifié ayant de 1 à 6 atomes de carbone ou un radical de formule

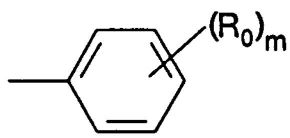

dans lequel $R_0$ et m ont la signification donnée précédemment,

. un radical de type spiro de formule :

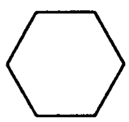

ou

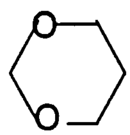

ou

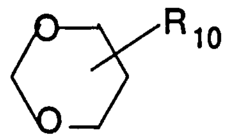

dans laquelle $R_{10}$ représentant un ou plusieurs radicaux alkyle, linéaires ou ramifiés ayant de 1 à 6 atomes de carbone.

**10.** Procédé selon l'une des revendications 1 à 6 caractérisé par le fait que le composé cétonique répond à la formule (Ia) dans laquelle A est le reste d'un composé carbocyclique monocyclique, saturé portant un ou plusieurs substituants tels que :

. un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,

. un radical alkyle ayant de 1 à 15 atomes de carbone porteur d'un groupe fonctionnel, de préférence une fonction OH, CN, $N[R_2]_2$, $COOR_2$ avec $R_2$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2.CH_2-CO-(CH_2)_4-CH_3$,

. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule $-C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,

. un radical $=C(CH_3)_2$ ou $=CH-(CH_2)_2-CH_3$,

. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_2$ dans laquelle $R_2$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,

. un radical alkoxy linéaire ou ramifié ayant de 1 à 6 atomes de carbone, de préférence un radical méthoxy,

éthoxy,

. deux atomes successifs du cycle peuvent être reliés entre eux par un pont époxy ou par un pont tel que les radicaux méthylène dioxy, éthylène dioxy, propylène dioxy,

. un radical de type spiro de formule

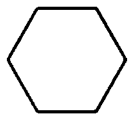 ou 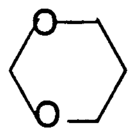 ou 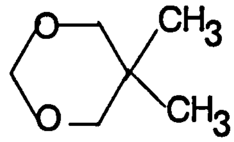

. un radical de formule

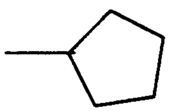 ou 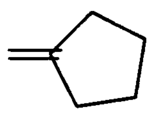 ou 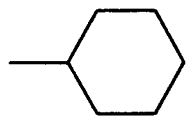 ou 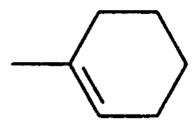

. un radical de formule

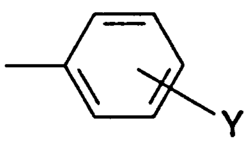 ou 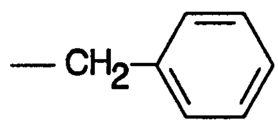 ou 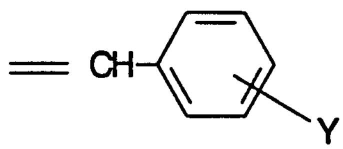

Y représentant un atome d'hydrogène, un atome d'halogène, de préférence de fluor ou de chlore,

. un radical de formule

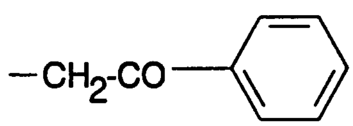 ou 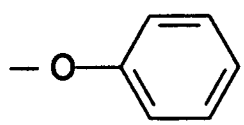

. un groupe OH,

. un groupe $COOR_4$, $R_4$ représentant un atome d'hydrogène ou un radical alkyle ayant de 1 à 4 atomes de carbone, de préférence méthyle, éthyle,

. un groupe CN,

. un atome d'halogène, de préférence, de fluor, de chlore, de brome.

**11.** Procédé selon l'une des revendications 1 à 6 caractérisé par le fait que le composé cétonique répond à la formule (Ia) dans laquelle A est le reste d'un composé carbocyclique monocyclique, insaturé portant un ou plusieurs substituants tels que :

. un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,

. un radical alkyle ayant de 1 à 15 atomes de carbone porteur d'un groupe fonctionnel, de préférence une fonction OH, CN, $N[R_2]_2$, $COOR_2$ avec $R_2$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical

de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2-CH_2-CO-(CH_2)_4-CH_3$,

. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule _$C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,
. un radical $=C(CH_3)_2$ ou $=CH-(CH_2)_2-CH_3$,
. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_2$ dans laquelle $R_2$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,
. un radical alkoxy linéaire ou ramifié ayant de 1 à 6 atomes de carbone, de préférence un radical méthoxy, éthoxy,
. un radical de formule

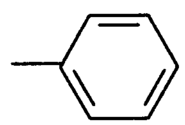

. un groupe OH,
. un groupe COOR, R représentant un atome d'hydrogène ou un radical alkyle ayant de 1 à 4 atomes de carbone,de préférence méthyle, éthyle,
. un atome d'halogène, de préférence, de fluor, de chlore, de brome.

**12.** Procédé selon l'une des revendications 1 à 5, 7 et 8 caractérisé par le fait que le composé cétonique répond à la formule (Ia) dans laquelle A est le reste d'un composé carbocyclique polycyclique, de préférence bicylique comprenant deux carbocycles saturés portant un ou plusieurs substituants tels que :

. un radical alkyle linéaire ou ramifié ayant de 1 à 6 atomes de carbone, de préférence un radical méthyle, isopropyle,
. un radical de formule $-CH_2Br$,
. un radical alkoxy linéaire ou ramifié ayant de 1 à 6 atomes de carbone, de préférence un radical méthoxy,
. un radical de formule $=CH_2$,
. un groupe OH,
. un groupe -COOH,
. un atome d'halogène, de préférence, de fluor, de chlore, de brome,
. un groupe $CF_3$

**13.** Procédé selon l'une des revendications 1 à 5, 7 et 8 caractérisé par le fait que le composé cétonique répond à la formule (Ia) dans laquelle A est le reste d'un composé carbocyclique bicylique comprenant deux carbocycles dont l'un est saturé, et l'autre insaturé, portant un ou plusieurs substituants tels que :

. un radical alkyle linéaire ou ramifié ayant de 1 à 6 atomes de carbone, de préférence un radical méthyle,
. un radical de formule

-C=CH2,
I
CH3

. un radical de formule $-CH_2-O-CH_3$,
. un atome d'halogène, de préférence, de chlore.

**14.** Procédé selon l'une des revendications 1 à 5, 7 et 8 caractérisé par le fait que le composé cétonique répond à la formule (Ia) dans laquelle A est le reste d'un composé carbocyclique polycylique, de préférence, bicylique comprenant deux carbocycles insaturés, portant un ou plusieurs radicaux alkyle.

**15.** Procédé selon l'une des revendications 1 à 5, 7 et 8 caractérisé par le fait que le composé cétonique répond à la formule (Ia) dans laquelle A est le reste d'un composé carbocyclique polycylique comprenant au moins un carbocycle aromatique, de préférence, un cycle benzénique, portant un ou plusieurs substituants tels que :

. un radical alkyle linéaire ou ramifié ayant de 1 à 6 atomes de carbone, de préférence un radical méthyle, tert-butyle,
. un radical de formule

=C-CN,
I
CN

. un radical alkoxy linéaire ou ramifié ayant de 1 à 6 atomes de carbone, de préférence un radical méthoxy,
. un radical alkoxy linéaire ou ramifié ayant de 1 à 6 atomes de carbone porteur d'autres groupes fonctionnels tels que, par exemple, un groupe OH et/ou $N[R_2]_2$ avec $R_2$, identiques ou différents, représentant un atome d'hydrogène, un radical alkyle linéaire ou ramifié ayant de 1 à 6 atomes de carbone, de préférence un radical de formule $-O-CH_2-CHOH-CH_2-NHBu-t$
. un groupe OH,
. un groupe acyle ayant de 2 à 6 atomes de carbone, de préférence un radical acétyle ou de formule -CO-tert-butyle,
. un groupe $-CH_2-COOH$,
. un groupe $-NH_2$,
. un atome d'halogène, de préférence, de fluor, de chlore, de brome.

**16.** Procédé selon la revendication 1 caractérisé par le fait que le composé cétonique répond à la formule qénérale (Ib) :

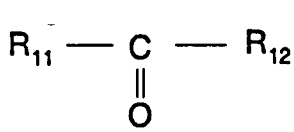

(Ib)

dans ladite formule (Ib) :

- $R_{11}$ et $R_{12}$, identiques ou différents représentent un radical hydrocarboné ayant de 1 à 20 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique ; un radical aliphatique saturé ou insaturé, linéaire ou ramifié, porteur d'un substituant cyclique.
- au moins l'un des radicaux $R_{11}$ et $R_{12}$ comprend un atome de carbone en position $\alpha$ par rapport au groupe carbonyle qui porte au moins un atome d'hydrogène ou une fonction ester.

**17.** Procédé selon la revendication 16 caractérisé par le fait que le composé cétonique répond à la formule générale (Ib) dans laquelle $R_{11}$ et $R_{12}$ représentent :

- un radical aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ; la chaîne hydrocarbonée pouvant être éventuellement interrompue par l'un des groupes tels que par exemples les groupes précités Z ou et/ou porteuses de l'un des substituants suivants précédemment définis : - OH ; -CN ; $-N[R_2]_2$;-$COOR_2$ ; $-CF_3$ ou -X,
- un radical aliphatique acyclique, saturé ou insaturé, linéaire ou ramifié porteur d'un substituant cyclique.

**18.** Procédé selon la revendication 16 caractérisé par le fait que le composé cétonique répond à la formule générale (Ib) dans laquelle l'un des radicaux $R_{11}$ ou $R_{12}$ représente un radical hydrocarboné cyclique, saturé ou non ayant de préférence 5 ou 6 atomes de carbone dans le cycle ; un radical hétérocyclique, saturé ou non, comportant notamment 5 ou 6 atomes dans le cycle dont 1 ou 2 hétéroatomes tels que les atomes d'azote, de soufre et d'oxygène ; un radical hydrocarboné aromatique, monocyclique ou polycyclique condensé ou non.

**19.** Procédé selon la revendication 1 à 18 caractérisé par le fait que le composé cétonique répondant à la formule (Ia) est la cylopentanone et/ou la 2-méthylcyclopentanone ou la 2-méthyl-2-carboxyméthylcyclopentanone, la 2-[(p-chloro)benzylidène]cyclopentanone ou la 2-méthyl-2-carboxyméthyl-5-[(p-chloro)benzylidène]cyclopentanone.

**20.** Procédé selon l'une des revendications 1 à 20 caractérisé par le fait que l'agent d'alkylation est choisi parmi les esters organiques, les dialkylsulfates, les alcools.

**21.** Procédé selon la revendication 20 caractérisé par le fait que l'agent d'alkylation est choisi parmi les esters organiques répondant à la formule générale (II) :

$$[R_a - CO - O - ]_p - R_b \qquad (II)$$

dans laquelle :

- $R_a$ représente :
  - . un radical $R_c$ qui peut être un radical hydrocarboné ayant de 1 à 12 atomes de carbone qui peut être un radical aliphatique saturé ou insaturé, linéaire ou ramifié, éventuellement porteur d'un atome d'halogène, de préférence, un atome de chlore ; un radical carbocyclique saturé, insaturé ou aromatique, monocyclique ou polycyclique ; un radical aliphatique acyclique, saturé ou insaturé, linéaire ou ramifié porteur d'un substituant cyclique,
  - . un radical $R_d$ - O - CO - $R_f$ - dans lequel le radical $R_d$ a la signification donnée pour le radical $R_c$ et le radical $R_f$ représente un lien valentiel ou un radical divalent aliphatique, saturé ou insaturé, linéaire ou ramifié ayant au moins 1 atome de carbone,
  - . un radical $R_d$ - O - dans lequel le radical $R_d$ a la signification donnée pour le radical $R_c$,
  - . un radical $R_d$ - O - CO - O - dans lequel le radical $R_d$ a la signification donnée pour le radical $R_c$,
- $R_b$ représente :
  - . un radical $R_c$ tel que précédemment défini,
  - . un métal alcalin ou alcalino-terreux,
- $R_a$ et $R_b$ peuvent former ensemble un radical divalent aliphatique saturé ou insaturé, linéaire ou ramifié, ayant au moins 2 atomes de carbone.
- p est un nombre entier égal à 1 ou 2.

**22.** Procédé selon la revendication 20 caractérisé par le fait que l'agent d'alkylation est choisi parmi les esters d'acides carboxyliques de formule (IIa) :

$$R_a - CO - O - R_b \qquad (IIa)$$

dans laquelle :

- $R_a$ représente :

  . un radical alkyle linéaire ou ramifié ayant de 1 à 6 atomes de carbone, de préférence, de 1 à 3 atomes de carbone,
  . un radical cycloalkyle ayant 5 ou 6 atomes de carbone, de préférence, 6 atomes de carbone,
  . un radical aralkyle ayant de 7 à 12 atomes de carbone,
  . un radical $R_c$ - O - CO - $R_d$ - dans lequel le radical $R_c$ a la signification donnée ci-dessus pour le radical $R_a$ et le radical divalent $R_d$ représente un lien valentiel ou un radical alkylène ayant de 1 à 6 atomes de carbone.

- $R_b$ représente :

  . un radical alkyle, linéaire ou ramifié ayant de 1 à 6 atomes de carbone,
  . un radical cycloalkyle ayant 5 ou 6 atomes de carbone,

- $R_a$ et $R_b$ peuvent former ensemble un radical alkylène ayant de 2 à 4 atomes de carbone.

**23.** Procédé selon la revendication 20 caractérisé par le fait que l'agent d'alkylation est choisi parmi les carbonates de formule générale (IIb) :

$$[R_a - O - CO - O -]_p - R_b \qquad (IIb)$$

dans laquelle :

- $R_a$ représente :

  . un radical alkyle, linéaire ou ramifié, ayant 1 à 6 atomes de carbone et éventuellement porteur d'un atome d'halogène, de préférence, un atome de chlore,
  . un radical cycloalkyle ayant 5 à 6 atomes de carbone,
  . un radical aryle ayant de 6 à 12 atomes de carbone,
  . un radical $R_d$ - O - CO - dans lequel $R_d$ représente un radical alkyle, linéaire ou ramifié ayant 1 à 6 atomes de carbone, un radical cycloalkyle ayant 5 ou 6 atomes de carbone ;

- $R_b$ représente :

  . un radical alkyle, linéaire ou ramifié, ayant 1 à 6 atomes de carbone,
  . un radical cycloalkyle ayant 5 ou 6 atomes de carbone,
  . un métal alcalin ou alcalino-terreux, de préférence le sodium ou le potassium,
  p = 1 ou p = 2 lorsque $R_b$ représente un métal alcalino-terreux ;

- $R_a$ et $R_b$ peuvent former ensemble un radical alkylène ayant 2 à 6 atomes de carbone.

**24.** Procédé selon la revendication 20 caractérisé par le fait que l'agent d'alkylation est choisi parmi les dialkylsulfates de formule $R_g$-O-$SO_2$-O-$R_h$, dans ladite formule, $R_g$ et $R_h$, identiques ou différents représentent un radical alkyle, linéaire ou ramifié, ayant de 1 à 6 atomes de carbone.

**25.** Procédé selon la revendication 20 caractérisé par le fait que l'agent d'alkylation est choisi parmi les alcools répondant à la formule (IIc) :

$$R_i - OH \qquad (IIc)$$

dans ladite formule, $R_i$ représente un radical hydrocarboné ayant de 1 à 12 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

**26.** Procédé selon l'une des revendications 20 à 25 caractérisé par le fait que l'agent d'alkylation est choisi parmi : le diméthylsulfate ; le diméthylcarbonate, le chlorométhylméthylcarbonate ; l'acétate de méthyle ou l'acétate d'éthyle ; le méthanol ou l'éthanol.

**27.** Procédé selon l'une des revendications 1 à 26 caractérisé par le fait que le catalyseur est un phosphate métallique ou d'ammonium tel que dihydrogénophosphate, monohydrogénophosphate, phosphate, phosphate condensé, de préférence, pyrophosphate ou polyphosphate.

**28.** Procédé selon la revendication 27 caractérisé par le fait que le catalyseur est un orthophosphate d'un élément du groupe 1a, 2a, 3a ou 3b, de préférence du groupe 3a.

**29.** Procédé selon l'une des revendications 27 et 28 caractérisé par le fait que le catalyseur mis en oeuvre est choisi parmi les phosphates, les pyrophosphates, les tripolyphosphates ou les pentapolyphosphate de sodium ou de potassium.

**30.** Procédé selon l'une des revendications 27 et 28 caractérisé par le fait que le catalyseur de l'invention répond à la formule suivante :

$$MPO_4 \qquad (III)$$

dans laquelle :

- M représente une terre rare trivalente à savoir un lanthanide ayant un numéro atomique de 57 à 71 et l'yttrium ainsi que le scandium.

**31.** Procédé selon l'une des revendications 27 et 28 caractérisé par le fait que le catalyseur de l'invention répond à la formule suivante :

$$MPO_4\,(Im)_p \qquad (IIIa)$$

dans laquelle :

- M représente $M_3$ une terre rare trivalente ou un mélange d'au moins une terre trivalente $M_3$ et d'au moins un élément choisi parmi les métaux alcalins $M_1$ et les métaux alcalino-terreux $M_2$ avec la relation :

$$M = \alpha M_1^{\ +} + \beta M_2^{\ ++} + \gamma M_3^{\ 3+} \text{ et } \alpha + 2\beta + 3\gamma = 3$$

- Im correspond à un composé d'imprégnation basique constitué de métal alcalino-terreux, de préférence de métal alcalin et leurs mélanges associés à un contre-anion pour assurer la neutralité électrique.
- $\alpha$ est un coefficient compris entre 0 et 3, avantageusement supérieur à 0,01 et au plus égal à 0,5, de préférence compris entre 0,05 et 0,2.
- $\beta$ est un coefficient compris entre 0 et 3/2, de préférence entre 0 et 1/3 ou bien 1 $\pm$ 0,1.
- $\gamma$ est un coefficient compris entre 0 et 1, avantageusement au moins égal à 1/3, de préférence à 1/2,
- p est inférieur à 0,5 et avantageusement compris entre 0,04 et 0,25.

**32.** Procédé selon la revendication 31 caractérisé par le fait que le catalyseur de l'invention répond à la formule (IIIa) dans laquelle :

- $M_1$ est choisi dans le groupe des éléments de la colonne 1a et leurs mélanges, de préférence les métaux alcalins tels que le lithium, le sodium, le potassium, le rubidium et le césium,
- $M_2$ est choisi dans le groupe des éléments de la colonne 2a et leurs mélanges, de préférence les métaux alcalino-terreux tels que le béryllium, le magnésium, le calcium, le strontium et le baryum,
- $M_3$ est choisi dans le groupe des terres rares trivalentes choisies parmi les lanthanides, l'yttrium le scandium et leurs mélanges, de préférence les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium.

**33.** Procédé selon l'une des revendications 30 à 32 caractérisé par le fait que le catalyseur de l'invention est un orthophosphate de métal de terre rare trivalente, de préférence de terre rare cérique, éventuellement dopé par un métal alcalin et/ou alcalino-terreux.

**34.** Procédé selon l'une des revendications 30 à 33 caractérisé par le fait que le catalyseur de l'invention répond à la

formule (IIIa) dans laquelle M est le lanthane, le cérium et le samarium dopé par un métal alcalin, de préférence le césium.

**35.** Procédé selon l'une des revendications 30 à 34 caractérisé par le fait que le catalyseur de l'invention a une teneur en agent dopant telle que le pourcentage pondéral de l'agent dopant par rapport au phosphate de terre trivalente est compris entre 0 et 25 %, de préférence entre 3 et 15%.

**36.** Procédé selon la revendication 30 caractérisé par le fait que le composé cétonique de départ mis à réagir avec un agent d'alkylation présente au moins un atome d'hydrogène en position $\alpha$ par rapport au groupe carbonyle.

**37.** Procédé selon l'une des revendications 27 à 36 caractérisé par le fait que le composé cétonique de départ mis à réagir présente au moins une fonction ester en position $\alpha$ par rapport au groupe carbonyle.

**38.** Procédé selon l'une des revendications 30 à 37 caractérisé par le fait que la quantité d'agent d'alkylation est telle que le rapport entre le nombre de moles de l'agent d'alkylation et le nombre d'atomes d'hydrogène remplacés par un groupe alkyle d'un composé cétonique varie entre 0,5 et 500, de préférence entre 1 et 10.

**39.** Procédé selon l'une des revendications 1 à 38 caractérisé par le fait la productivité pondérale et horaire du catalyseur est comprise entre 0,1 et 20 $h^{-1}$, de préférence entre 1 et 5 $h^{-1}$.

**40.** Procédé selon l'une des revendications 1 à 39 caractérisé par le fait que la température de la réaction de C-alkylation est comprise entre 80°C et 500°C, de préférence entre 200°C et 350°C.

**41.** Procédé selon l'une des revendications 1 à 40 caractérisé par le fait que la réaction est conduite en phase vapeur.

**42.** Procédé selon l'une des revendications 1 à 40 caractérisé par le fait que la réaction est conduite en phase liquide, éventuellement en présence d'un solvant organique.

**Claims**

**1.** A process for the C-alkylation of a ketone compound having at least one hydrogen atom or ester group in the $\alpha$ position in relation to the carbonyl group, characterised in that the alkylation reaction is carried out in the $\alpha$ position in relation to the carbonyl group, in the presence of an effective quantity of a catalyst comprising a condensed or uncondensed orthophosphate anion.

**2.** A process according to Claim 1, characterised in that a ketone compound which has at least one hydrogen atom in the $\alpha$ position from the carbonyl group is reacted with an alkylation agent, in the presence of said catalyst.

**3.** A process according to Claim 1, characterised in that a ketone compound having at least one ester group in the $\alpha$ position in relation to the carbonyl group is contacted with said catalyst ; the ester group preferably being a radical of the type - $COOR_e$, wherein $R_e$ is a linear or branched alkyl radical with 1 to 6 carbon atoms, and preferably with 1 to 4 carbon atoms, and more preferably a methyl or ethyl radical.

**4.** A process according to one of Claims 1 to 3, characterised in that the ketone compound corresponds to general formula (Ia):

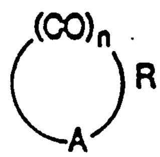

(Ia)

wherein:

- A represents the remainder of a cycle which forms all or part of a monocyclic or polycyclic system comprising at least one carbonyl group and having at least one hydrogen atom and an ester group in the $\alpha$ position in relation to the carbonyl group,

- R represents a hydrogen atom or one or more substituents, which may be identical or different,
- n is a number, preferably 1 or 2.

**5.** A process according to one of Claims 1 to 4, characterised in that the ketone compound corresponds to formula (Ia), wherein the remainder A which may be substituted represents the remainder of:

- a saturated or unsaturated, monocyclic or carbocyclic compound,
- a polycyclic compound comprising at least two saturated and/or unsaturated carbocycles,
- a polycyclic compound comprising at least two saturated and/or unsaturated cycles : one or more of the carbon atoms being able to be replaced by a heteroatom,
- a polycyclic compound comprising at least two carbocycles, one of which is aromatic.

**6.** A process according to one of Claims 1 to 5, characterised in that the ketone compound is a carbocyclic monocyclic compound with between 3 and 20 carbon atoms in the cycle, preferably with 5 or 6 carbon atoms; said carbocycle optionally comprising 1 to 2 double bonds.

**7.** A process according to one of Claims 1 to 5, characterised in that the ketone compound is a polycyclic compound with between 3 and 8 carbon atoms in each cycle, preferably 5 or 6 carbon atoms.

**8.** A process according to one of Claims 1 to 5, characterised in that the ketone compound is a polycyclic compound comprising at least two saturated and/or unsaturated cycles: one or two carbon atoms being able to be replaced by a heteroatom, preferably an oxygen or nitrogen atom.

**9.** A process according to one of Claims 1 to 8, characterised in that the ketone compound corresponds to formula (Ia), wherein the remainder A has one or more substituents R, such that:

- R representing $R_0$, one of the following groups:

  . a linear or branched acyclic aliphatic radical with 1 to 20 carbon atoms which may be saturated or which may comprise one or more unsaturated bonds on the chain, preferably 1 to 3 unsaturated bonds which are preferably simple or conjugated double bonds; the hydrocarbon chain possibly being able to be:
  . interrupted by one of the following groups represented by Z:
  $-O-$; $-CO-$; $COO-$; $-NR_2-$; $-CO-NR_2$; $-S-$; $-SO_2-$;
  in which formulae $R_2$ represents a hydrogen atom, a linear or branched alkyl radical with 1 to 6 carbon atoms,
  . and/or contains one of the following substituents:
  $-OH$; $-CN$; $-N[R_2]_2$; $-COOR_2$; $-CF_3$ or $-X$;
  in which formulae, the $R_2$ radicals which may be identical or different represent a hydrogen atom, a linear or branched alkyl radical with 1 to 6 carbon atoms, and X represents a halogen atom, preferably fluorine, chlorine, or bromine,
  . a $=R_3$ type radical, wherein $R_3$ represents an alkylidene radical with 1 to 6 carbon atoms, a radical of the formula $=C(CN)_2$ or a cycloalkylidene or cycloalkenylidene radical with 5 or 6 carbon atoms, or a benzylidene radical which may be substituted, preferably by a halogen atom X,
  . a linear or branched alkoxy radical with 1 to 6 carbon atoms,
  . two successive atoms of the cycle can be connected together by an epoxy bridge or by an alkylenedioxy type bridge having 1 to 4 carbon atoms, preferably methylene dioxy, ethylene dioxy, or propylene dioxy radicals,
  . a OH group,
  . a $COOR_4$ group, $R_4$ representing a hydrogen atom or a alkyl radical with 1 to 4 carbon atoms, preferably methyl or ethyl,
  . a CN group,
  . a halogen atom, preferably fluorine, chlorine or bromine.
  . a $-CF_3-$ group.

- R representing $R_1$, one of the following more complex groups:

  . a saturated or unsaturated carbocyclic radical with 4 to 7 carbon atoms, preferably a cyclopentyl, cyclohexyl, cyclopentene-2-yl, cyclopentene-3-yl, cyclohexene-1-yl, cyclohexene-2-yl, cyclohexene-3-yl,
  . a radical of the formula

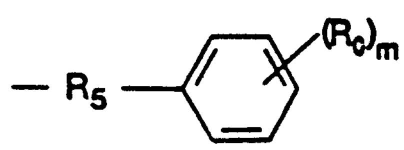

wherein $R_5$ represents a valence bond or a saturated or unsaturated, linear or branched, divalent hydrocarbon radical with 1 to 6 carbon atoms, such as methylene, ethylene, propylene, isopropylene, isopropylidene, and $R_0$ having the meaning given hereinabove, and m is a whole number from 0 to 4,

. a - $R_5$ - Z - $R_8$ radical, wherein Z and $R_5$ have the meaning given hereinabove, $R_8$ representing a linear or branched alkyl radical with 1 to 6 carbon atoms, or a radical of the formula

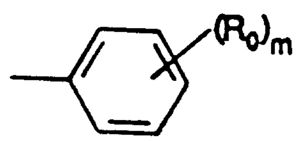

wherein $R_0$ and m have the meaning given hereinabove,

. a spiro type radical of the formula

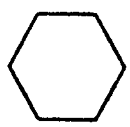

or

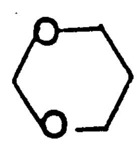

or

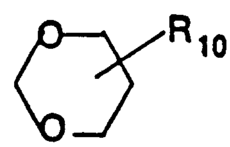

wherein $R_{10}$ represents one or more linear or branched alkyl radicals with 1 to 6 carbon atoms.

**10.** A process according to one of Claims 1 to 6, characterised in that the ketone compound corresponds to formula (Ia), wherein A is the remainder of a saturated, monocyclic, carbocyclic compound with one or more substituents, such as:

. a linear or branched alkyl radical with 1 to 15 carbon atoms, preferably a methyl, ethyl, n-propyl, isopropyl, n-butyl, sec-butyl, tert-butyl, n-pentyl, isopentyl, tert-pentyl, n-hexyl, or n-heptyl radical,

. an alkyl radical with 1 to 15 carbon atoms with a functional group, preferably a OH, CN, $N[R_2]_2$, $COOR_2$ group with $R_2$ which can be identical or different representing a hydrogen atom, or a linear or branched alkyl radical with 1 to 6 carbon atoms: the alkyl chain being able to be interrupted by an oxygen atom or a carbonyl, carboxy or amino group which may be substituted, in particular a radical of the formula $-NHCH_3$ or $-N(CH_3)_2$, a radical of the formula $-CH_2-CH_2-CN$, a radical of the formula $-CH_2-CO-(CH_2)_4-COOH$, a radical of the formula $COCH(CH_3)_2$, a radical of the formula $-(CH_2)_6-COOH$, a radical of the formula $-CH_2-COOCH_3$, a radical of the formula $-CH_2-COOC_2H_5$, a radical of the formula $-CH_2-CH_2-COOCH_3$, a radical of the formula $-(CH_2)_6-COOCH_3$, a radical of the formula $-(CH_2)_6-COOC_2H_5$, a radical of the formula $-(CH_2)_5-COOC_2H_5$, a radical of the formula $-CO-CH_3$, a radical of the formula $-C(CH_3)_2-CO-CH_3$, a radical of the formula $-CH_2-CH_2-CO-(CH_2)_4-CH_3$,

. a linear or branched aklenylene or alkylidene radical comprising one or two double bonds with 1 to 15 carbon atoms, preferably a radical of the formula $-CH_2-CH=CH_2$, a radical of the formula $-C(CH_3)=CH_2$, a radical of the formula $-CH_2-CH=CH-C_2H_5$, a radical of the formula $-CH_2-CH = CH-(CH_2)_2 - CH_3$, a radical of the formula $-CH=CH-(CH_2)_4-CH_3$, a radical of the formula $-CH_2-CH=C(CH_3)_2$, a radical of the formula $-CH_2-CH=CCH_3-(CH_2)_2-CH=C(CH_3)_2$,

. a $=C(CH_3)_2$ or $=CH-(CH_2)_2-CH_3$ radical,

. a linear or branched alkeneylene or alkylidene radical comprising one or two double bonds with 1 to 15 carbon atoms and which carries a functional group, preferably a OH, CN, $NH_2$, $COOR_2$ group, wherein $R_2$ has the meaning given hereinabove: the unsaturated chain being able to be interrupted by a oxygen atom, a carbonyl group or a carboxy group, more particularly a radical of the formula $-CH_2-CH=CH-(CH_2)_3-COOH$, a radical of

the formula $-CH=CH-C(CH_3)=CH-COOH$, a radical of the formula $-CH_2-CH=CH-(CH_2)_3-COOCH_3$, a radical of the formula $-CH=CH-C(CH_3)=CH-COOCH_3$, a radical of the formula $-CH=CH-CO-CH_3$, a radical of the formula $-CH=CH-CO-(CH_2)_4-CH_3$, or a radical of the formula $-CH=CH-CHOH-(CH_2)_4-CH_3$,

. a linear or branched alkoxy radical with 1 to 6 carbon atoms, preferably a methoxy or ethoxy radical,

. two successive atoms of the cycle being able to be connected together by an epoxy bridge or by a bridge such as a methylene dioxy, ethylene dioxy, or propylene dioxy radical,

. a spiro type radical of the formula

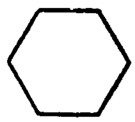

or

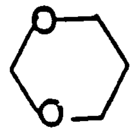

or

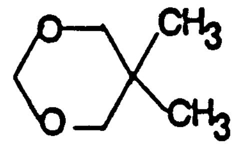

. a radical of the formula

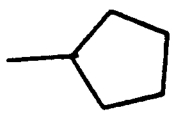

or

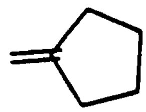

or

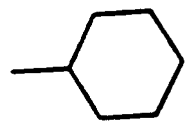

or

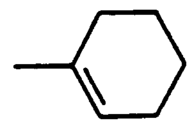

. a radical of the formula

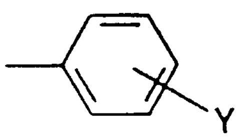

or

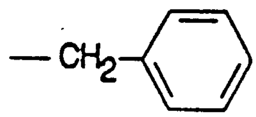

or

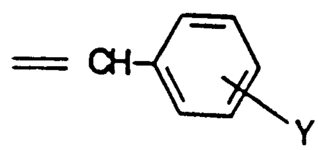

Y representing a hydrogen atom, or a halogen atom, preferably fluorine or chlorine,

. a radical of the formula

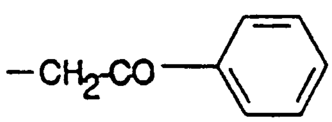

or

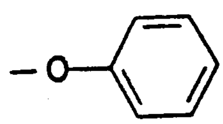

. a OH group,

. a $COOR_4$ group where $R_4$ represents a hydrogen atom or an alkyl radical with 1 to 4 carbon atoms, preferably methyl or ethyl,

. a CN group,

. a halogen atom, preferably fluorine, chlorine, or bromine.

**11.** A process according to one of Claims 1 to 6, characterised in that the ketone compound corresponds to formula (Ia), wherein A is the remainder of an unsaturated, monocyclic, carbocyclic compound bearing one or more substituents, such as:

. a linear or branched alkyl radical with 1 to 15 carbon atoms, preferably a methyl, ethyl, n-propyl, isopropyl, n-butyl, sec-butyl, tert-butyl, n-pentyl, isopentyl, tert-pentyl, n-hexyl, n-heptyl radical,

. an alkyl radical having 1 to 15 carbon atoms and bearing a functional group, preferably a OH, CN, $N[R_2]_2$, $COOR_2$ function, where $R_2$ which can be identical or different represents a hydrogen atom, a linear or branched alkyl radical with 1 to 6 carbon atoms: the alkyl chain being able to be interrupted by an oxygen atom or a carbonyl, carboxy or amino group which may be substituted, in particular a radical of the formula $-NHCH_3$ or $-N(CH_3)_2$, a radical of the formula $-CH_2-CH_2-CN$, a radical of the formula $-CH_2-CO-(CH_2)_4-COOH$, a radical of the formula $COCH(CH_3)_2$, a radical of the formula $-(CH_2)_6-COOH$, a radical of the formula $-CH_2-COOCH_3$, a radical of the formula $-CH_2-COOC_2H_5$, a radical of the formula $-CH_2-CH_2-COOCH_3$, a radical of the formula-$(CH_2)_6-COOCH_3$, a radical of the formula - $(CH_2)_6-COOC_2H_5$, a radical of the formula $-(CH_2)_5-COOC_2H_5$, a radical of the formula - $CO-CH_3$, a radical of the formula $-C(CH_3)_2-CO-CH_3$, a radical of the formula - $CH_2-CH_2-CO-(CH_2)_4-CH_3$,

. a linear or branched alkylidene or alkenylene radical comprising one or two double bonds having 1 to 15 carbon atoms, preferably a radical of the formula - $CH_2-CH=CH_2$, a radical of the formula _$C(CH_3)=CH_2$, a radical of the formula - $CH_2-CH=CH-C_2H_5$, a radical of the formula $-CH_2-CH=CH-(CH_2)_2-CH_3$, a radical of the formula - $CH=CH-(CH_2)_4-CH_3$, a radical of the formula $-CH_2-CH=C(CH_3)_2$, a radical of the formula - $CH_2-CH=CCH_3-(CH_2)_2-CH=C(CH_3)_2$,

. a $=C(CH_3)_2$ or $=CH-(CH_2)_2-CH_3$ radical,

. a linear or branched alkenylene or alkylidene radical with one or two double bonds, having 1 to 15 carbon atoms and bearing a functional group, preferably a OH, CN, $NH_2$, or $COOR_2$ function, wherein $R_2$ has the meaning given hereinabove: the unsaturated chain being able to be interrupted by an oxygen atom, a carbonyl or carboxy group, in particular a radical of the formula $-CH_2-CH=CH-(CH_2)_3-COOH$, a radical of the formula - $CH-CH-C(CH_3)=CH-COOH$, a radical of the formula - $CH_2-CH=CH-(CH_2)_3-COOH_3$, a radical of the formula - $CH=CH-C(CH_3)=CH-COOCH_3$, a radical of the formula $-CH=CH-CO-CH_3$, a radical of the formula - $CH=CH-CO-(CH_2)_4-CH_3$, or a radical of the formula $-CH=CH-CHOH-(CH_2)_4-CH_3$,

. a linear or branched alkoxy radical with 1 to 6 carbon atoms, preferably a methoxy or epoxy radical,

. a radical of the formula

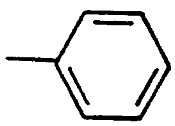

. a OH group,

. a COOR group, R representing a hydrogen atom or an alkyl radical with 1 to 4 carbon atoms, preferably methyl or ethyl,

. a halogen atom, preferably fluorine, chlorine or bromine.

**12.** A process according to one of Claims 1 to 5, 7 and 8, characterised in that the ketone compound corresponds to formula (Ia), wherein A is the remainder of a carbocylic compound which is polycyclic and preferably bicyclic, comprising two saturated carbocycles bearing one or more substituents, such as:

. a linear or branched alkyl radical with 1 to 6 carbon atoms, preferably a methyl or isopropyl radical,

. a radical of the formula $-CH_2Br$,

. a linear or branched alkoxy radical with 1 to 6 carbon atoms, preferably a methoxy radical,

. a radical of the formula $=CH_2$,

. a OH group,

. a -COOH group,

. a halogen atom, preferably fluorine, chlorine or bromine,

. a $CF_3$ group.

**13.** A process according to one of Claims 1 to 5, 7 and 8, characterised in that the ketone compound corresponds to formula (Ia), wherein A is the remainder of a bicyclic carbocylic compound comprising two carbocycles, one of which is saturated, and the other of which is unsaturated, bearing one or more substituents, such as:

. a linear or branched alkyl radical with 1 to 6 carbon atoms, preferably a methyl radical,

. a radical of the formula

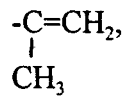

- a radical of the formula $-CH_2-O-CH_3$,
- a halogen atom, preferably chlorine.

**14.** A process according to one of Claims 1 to 5, 7 and 8, characterised in that the ketone compound corresponds to formula (Ia), wherein A is the remainder of a carbocyclic compound which is polycyclic and preferably bicyclic comprising two unsaturated carbocycles bearing one or more alkyl radicals.

**15.** A process according to one of Claims 1 to 5, 7 and 8, characterised in that the ketone compound corresponds to formula (Ia), wherein A is the remainder of a polycyclic carbocyclic compound comprising at least one aromatic carbocycle, preferably a benzene ring, bearing one or more substituents, such as:

- a linear or branched radical with 1 to 6 carbon atoms, preferably a methyl or tert-butyl radical,
- a radical of the formula

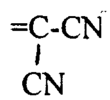

- a linear or branched alkoxy radical with 1 to 6 carbon atoms, preferably a methoxy radical,
- a linear or branched alkoxy radical with 1 to 6 carbon atoms bearing other functional groups, such as a OH group and/or $N[R_2]_2$ group with $R_2$ which may be identical or different representing a hydrogen atom, or a linear or branched alkyl radical with 1 to 6 carbon atoms, preferably a radical of the formula $-O-CH_2-CHOH-CH_2-NHBu-t$
- a OH group,
- a acyl group with 2 to 6 carbon atoms, preferably an acetyl radical or a radical of the formula -CO-tert-butyl,
- a $-CH_2-COOH$ group,
- a $-NH_2$ group,
- a halogen atom, preferably fluorine, chlorine, or bromine.

**16.** A process according to Claim 1, characterised in that the ketone compound corresponds to the general formula (Ib):

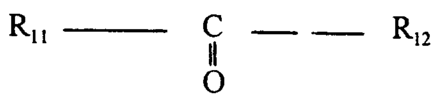

(Ib)

in which formula (Ib):

- $R_{11}$ and $R_{12}$ which are identical or different represent a hydrocarbon radical with 1 to 20 carbon atoms which can be a linear or branched, saturated or unsaturated, acyclic, aliphatic radical; a monocyclic or polycyclic, saturated, unsaturated or aromatic, heterocyclic or carbocyclic radical; a linear or branched, saturated or unsaturated aliphatic radical bearing a cyclic substituent.
- at least one of the $R_{11}$ and $R_{12}$ radicals comprises a carbon atom in the $\alpha$ position in relation to the carbonyl group which carries at least one hydrogen atom or an ester function.

**17.** A process according to Claim 16, characterised in that the ketone compound corresponds to the general formula (Ib), wherein $R_{11}$ and $R_{12}$ represent:

- a linear or branched acyclic aliphatic radical, preferably having 1 to 12 carbon atoms, which is saturated or which comprises one or more unsaturated bonds in the chain, usually 1 to 3 unsaturated bonds which can be simple or conjugated double bonds; the hydrocarbon chain being able to be interrupted by one of the groups, such as the afore-mentioned Z groups, and/or bearing one of the following substituents as defined hereinabove: - OH; -CN; -N[$R_2$]$_2$; - $COOR_2$; -$CF_3$ or -X,
- a linear or branched, saturated or unsaturated, acyclic aliphatic radical bearing a cyclic substituent.

**18.** A process according to Claim 16, characterised in that the ketone compound corresponds to general formula (Ib), wherein one of the $R_{11}$ or $R_{12}$ radicals represents a cyclic hydrocarbon radical which is saturated or unsaturated, and preferably having 5 or 6 carbon atoms in the cycle; a heterocyclic radical which may or may not be saturated and which comprises, in particular, 5 or 6 atoms in the cycle, 1 or 2 of which are heteroatoms, such as nitrogen, sulphur and oxygen atoms; or a aromatic, monocyclic or polycyclic hydrocarbon which may or may not be condensed.

**19.** A process according to Claims 1 to 18, characterised in that the ketone compound corresponding to formula (Ia) is cyclopentone and/or 2-methylcyclopentanone or 2-methyl-2-carboxymethylcyclopentanone, 2-[(p-chloro)benzylidene]cyclopentanone or 2-methyl-2-carboxymethyl-5-[(p-chloro)benzylidene]cyclopentanone.

**20.** A process according to one of Claims 1 to 20, characterised in that the alkylation agent is selected from organic esters, dialkylsulphates and alcohols.

**21.** A process according to Claim 20, characterised in that the alkylation agent is selected from organic esters corresponding to the general formula (II):

$$[ R_a - CO - O - ]_p - R_b \qquad \text{(II)}$$

wherein:

- $R_a$ represents:
  - . a $R_c$ radical which can be a hydrocarbon radical with 1 to 12 carbon atoms which can be a linear or branched, saturated or unsaturated aliphatic radical, possibly bearing a halogen atom, preferably a chlorine atom; a monocyclic or polycyclic, saturated, unsaturated or aromatic carbocyclic radical; a linear or branched acyclic aliphatic radical, bearing a cyclic substituent,
  - . a $R_d$ - O - CO - $R_f$ - radical, wherein the $R_d$ radical has the meaning given for the $R_c$ radical, and the $R_f$ radical represents a valence bond, or a linear or branched, saturated or unsaturated divalent aliphatic radical with at least 1 carbon atom,
  - . a $R_d$ - O - radical, wherein the $R_d$ radical has the meaning given for the $R_c$ radical,
  - . a $R_d$ - O - CO - O - radical, wherein the $R_d$ radical has the meaning given for the $R_c$ radical,
- $R_b$ represents:
  - . a $R_c$ radical as defined hereinabove,
  - . an alkali or alkaline-earth metal,
- $R_a$ and $R_b$ being able to form together a linear or branched, saturated or unsaturated, aliphatic divalent radical having at least 2 carbon atoms,
- p is a whole number equal to 1 or 2.

**22.** A process according to Claim 20, characterised in that the alkylation agent is selected from carboxylic acid esters of the formula (IIa):

$$R_a - CO - O - R_b \qquad \text{(IIa)}$$

wherein:

- $R_a$ represents:
  - . a linear or branched alkyl radical with 1 to 6 carbon atoms, preferably 1 to 3 carbon atoms,

. a cycloalkyl radical with 5 or 6 carbon atoms, preferably 6 carbon atoms,
. an aralkyl radical with 7 to 12 carbon atoms,
. a $R_c$ - O - CO - $R_d$ - radical, wherein the $R_c$ radical has the meaning given hereinabove for the $R_a$ radical, and the divalent $R_d$ radical represents a valence bond or an alkylene radical with 1 to 6 carbon atoms.

- $R_b$ represents:

. a linear or branched alkyl radical with 1 to 6 carbon atoms,
. a cycloalkyl radical with 5 or 6 carbon atoms,

- $R_a$ and $R_b$ being able to form together an alkylene radical with 2 to 4 carbon atoms.

**23.** A process according to Claim 20, characterised in that the alkylation agent is selected from carbonates of general formula (IIb):

$$[R_a - O - CO - O -]_p - R_b \qquad \text{(IIb)}$$

wherein:

- $R_a$ represents:

. a linear or branched alkyl radical with 1 to 6 carbon atoms, ad possibly bearing a halogen atom, preferably a chlorine atom,
. a cycloalkyl radical with 5 to 6 carbon atoms,
. an aryl radical with 6 to 12 carbon atoms,
. a $R_d$ - O - CO - radical, wherein $R_d$ represents a linear or branched, alkyl radical, with 1 to 6 carbon atoms, or a cycloalkyl radical having 5 to 6 carbon atoms;

- $R_b$ represents:

. a linear or branched alkyl radical with 1 to 6 carbon atoms,
. a cycloalkyl radical with 5 or 6 carbon atoms,
. an alkali or alkaline-earth metal, preferably sodium or potassium,
p = 1 or p = 2 when $R_b$ represents a alkaline-earth metal;

- $R_a$ and $R_b$ can form together a alkylene radical with 2 to 6 carbon atoms.

**24.** A process according to Claim 20, characterised in that the alkylation agent is selected from dialkylsulphates of the formula $R_g$-O-$SO_2$-O-$R_h$, in which formula $R_g$ and $R_h$ which may be identical or different represent a linear or branched alkyl radical with 1 to 6 carbon atoms.

**25.** A process according to Claim 20, characterised in that the alkylation agent is selected from alcohols corresponding to formula (IIc):

$$R_i - OH \qquad \text{(IIc)}$$

in which formula $R_i$ represents a hydrocarbon radical with 1 to 12 carbon atoms, which can be a linear or branched, saturated or unsaturated, acyclic aliphatic radical; a monocyclic or polycyclic, saturated or unsaturated cycloaliphatic radical; or a linear or branched, saturated or unsaturated aliphatic radical bearing a cyclic substituent.

**26.** A process according to one of Claims 20 to 25, characterised in that the alkylation agent is selected from: dimethylsulphate; dimethylcarbonate; chloromethyl methylcarbonate; methyl acetate or ethyl acetate; and methanol or ethanol.

**27.** A process according to one of Claims 1 to 26, characterized in that the catalyst is a metallic or ammonium phosphate, such as dihydrogen phosphate, monohydrogen phosphate, phosphate or condensed phosphate, preferably pyrophosphate or polyphosphate.

**28.** A process according to Claim 27, characterized in that the catalyst is an orthophosphate of an element from group 1a, 2a, 3a or 3b, preferably from group 3a.

**29.** A process according to either Claim 27 or Claim 28, characterized in that the catalyst used is selected from phosphates, pyrophosphates, tripolyphosphates or pentapolyphosphates of sodium or potassium.

**30.** A process according to either Claim 27 or Claim 28, characterised in that the catalyst of the invention corresponds to the following formula:

$$MPO_4 \qquad \text{(III)}$$

wherein:

- M represents a trivalent rare earth, namely a lanthanide with an atomic number of 57 to 71, yttrium and scadium.

**31.** A process according to either Claim 27 or Claim 28, characterized in that the catalyst of the invention corresponds to the following formula:

$$MPO_4\,(Im)_p \qquad \text{(IIIa)}$$

wherein:

- M represents a trivalent rare earth $M_3$, or a mixture of at least one trivalent rare earth $M_3$ and of at least one element selected from alkali metals $M_1$ and alkaline-earth metals $M_2$ in the relationship:

$$M = \alpha M_1^{\ +} + \beta M_2^{\ ++} + \gamma M_3^{\ 3+} \text{ and } \alpha + 2\beta + 3\gamma = 3$$

- Im corresponds to a basic impregnation compound constituted by an alkaline-earth metal, preferably an alkali metal and mixtures thereof associated with a counter-anion to provide electrical neutrality.
- $\alpha$ is a coefficient between 0 and 3, advantageously greater than 0.01 and at the most equal to 0.5, preferably between 0.05 and 0.2.
- $\beta$ is a coefficient between 0 and 3/2, preferably between 0 and 1/3, or $1 \pm 0.1$.
- $\gamma$ is a coefficient between 0 and 1, advantageously at least equal to 1/3, preferably to 1/2,
- p is less than 0.5, and advantageously between 0.04 and 0.25.

**32.** A process according to Claim 31, characterized in that the catalyst of the invention corresponds to formula (IIIa), wherein:

- $M_1$ is selected from the group of elements in column 1a and mixtures thereof, preferably alkali metals such as lithium, sodium, potassium, rubidium and cesium,
- $M_2$ is selected from the group of elements in column 2a and mixtures thereof, preferably alkaline-earth metals such as beryllium, magnesium, calcium, strontium and baryum,
- $M_3$ is selected from the group of trivalent rare earths selected from lanthanides, yttrium, scandium and mixtures thereof, preferably lanthanides, such as lanthanum, cerium, praseodymium, neodymium, samarium, europium, gadolinium, terbium, dysprosium, holmium, erbium, thulium, ytterbium and lutetium.

**33.** A process according to one of Claims 30 to 32, characterized in that the catalyst of the invention is an orthophosphate of a trivalent rare earth metal, preferably a ceric rare earth, which may be doped with an alkali and/or alkaline-earth metal.

**34.** A process according to one of Claims 30 to 33, characterized in that the catalyst of the invention corresponds to formula (IIIa), wherein M is lanthanum, cerium and samarium doped with an alkali metal, preferably cesium.

**35.** A process according to one of Claims 30 to 34, characterized in that the catalyst of the invention has a doping agent content such that the percentage by weight of doping agent in relation to the trivalent rare earth phosphate is between 0 and 25%, preferably between 3 and 15%.

36. A process according to Claim 30, characterized in that the initial ketone compound reacted with an alkylation agent has at least one hydrogen atom in the α position in relation to the carbonyl group.

37. A process according to one of Claims 27 to 36, characterised in that the initial ketone compound in the reaction has at least one ester group in the α position in relation to the carbonyl group.

38. A process according to one of Claims 30 to 37, characterized in that the quantity of alkylation agent is such that the ratio between the number of moles of alkylation agent and the number of hydrogen atoms replaced by an alkyl group of a ketone compound varies between 0.5 and 500, preferably between 1 and 10.

39. A process according to one of Claims 1 to 38, characterised in that the hourly productivity by weight of the catalyst is between 0.1 and 20 $h^{-1}$, preferably between 1 and 5 $h^{-1}$.

40. A process according to one of Claims 1 to 39, characterized in that the C-alkylation reaction temperature is between 80°C and 500°C, preferably between 200°C and 350°C.

41. A process according to one of Claims 1 to 40, characterized in that the reaction is carried out in vapour phase.

42. A process according to one of Claims 1 to 40, characterized in that the reaction is carried out in liquid phase, possibly in the presence of an organic solvent.

**Patentansprüche**

1. Verfahren zur C-Alkylierung einer Ketoverbindung mit mindestens einem Wasserstoffatom oder einer Esterfunktion in α-Position zur Carbonylgruppe, dadurch gekennzeichnet, daß man die Alkylierungsreaktion in α-Position zur Carbonylgruppe in Gegenwart einer wirksamen Menge eines Katalysators durchführt, der ein kondensiertes oder nichtkondensiertes Orthophosphatanion umfaßt.

2. Verfahren nach Anspruch 1, dadurch gekennzeichnet, daß man eine Ketoverbindung mit mindestens einem Wasserstoffatom in α-Position zur Carbonylgruppe in Gegenwart des Katalysators mit einem Alkylierungsreagenz reagieren läßt.

3. Verfahren nach Anspruch 1, dadurch gekennzeichnet, daß man eine Ketoverbindung mit mindestens einer Esterfunktion in α-Position zur Carbonylgruppe mit dem Katalysator in Kontakt bringt, wobei die Esterfunktion vorzugsweise ein Rest vom Typ $-COOR_e$ ist, wobei $R_e$ ein linearer oder verzweigter Alkylrest mit 1 bis 6 Kohlenstoffatomen, vorzugsweise mit 1 bis 4 Kohlenstoffatomen, insbesondere ein Methyl- oder Ethylrest, ist.

4. Verfahren nach einem der Ansprüche 1 bis 3, dadurch gekennzeichnet, daß die Ketoverbindung der allgemeinen Formel (Ia)

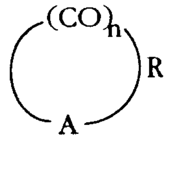

(Ia)

entspricht, in der:

- A den Rest eines Ringes symbolisiert, der als Ganzes oder zum Teil ein monocyclisches oder polycyclisches System ausbildet, das mindestens eine Carbonylgruppe umfaßt und mindestens ein Wasserstoffatom oder eine Esterfunktion in α-Position zur Carbonylgruppe aufweist;
- R ein Wasserstoffatom oder einen oder mehrere Substituenten, identisch oder verschieden, darstellt;
- n eine Zahl von vorzugsweise 1 oder 2 ist.

5. Verfahren nach einem der Ansprüche 1 bis 4, dadurch gekennzeichnet, daß die Ketoverbindung der Formel (Ia) entspricht, in der der gegebenenfalls substituierte Rest A folgende Reste darstellt:

- den Rest einer gesättigten oder ungesättigten, carbocyclischen monocyclischen Verbindung;

- den Rest einer polycyclischen Verbindung, die mindestens zwei gesättigte und/oder ungesättigte Carbocyclen umfaßt;
- den Rest einer polycyclischen Verbindung, die mindestens zwei gesättigte und/oder ungesättigte Ringe umfaßt, wobei eines oder mehrere der Kohlenstoffatome durch ein Heteroatom ersetzt werden kann;
- den Rest einer polycyclischen Verbindung, die mindestens zwei Carbocyclen umfaßt, von denen einer aromatisch ist.

**6.** Verfahren nach einem der Ansprüche 1 bis 5, dadurch gekennzeichnet, daß die Ketoverbindung eine carbocyclische monocyclische Verbindung mit einer Kohlenstoffatomanzahl im Ring zwischen 3 und 20 Kohlenstoffatomen, vorzugsweise von 5 oder 6 Kohlenstoffatomen, ist, wobei der Carbocyclus gegebenenfalls 1 bis 2 Doppelbindungen umfaßt.

**7.** Verfahren nach einem der Ansprüche 1 bis 5, dadurch gekennzeichnet, daß die Ketoverbindung eine polycyclische Verbindung mit einer Kohlenstoffanzahl in jedem Ring zwischen 3 und 8 Kohlenstoffatomen, vorzugsweise von 5 oder 6 Kohlenstoffatomen, ist.

**8.** Verfahren nach einem der Ansprüche 1 bis 5, dadurch gekennzeichnet, daß die Ketoverbindung eine polycyclische Verbindung ist, die mindestens zwei gesättigte und/oder ungesättigte Ringe umfaßt, wobei eines oder zwei der Kohlenstoffatome durch ein Heteroatom, vorzugsweise ein Sauerstoff-oder Stickstoffatom, ersetzt werden können.

**9.** Verfahren nach einem der Ansprüche 1 bis 8, dadurch gekennzeichnet, daß die Ketoverbindung der Formel (Ia) entspricht, in der der Rest A einen oder mehrere Substituenten R enthält, wobei:

- R den Rest $R_0$, eine der folgenden Gruppen, darstellt:

  - einen linearen oder verzweigten, acyclischen aliphatischen Rest mit 1 bis 20 Kohlenstoffatomen, der entweder gesättigt ist oder eine oder mehrere ungesättigte Bindungen in der Kette enthält, vorzugsweise 1 bis 3 ungesättigte Bindungen, die vorzugsweise einfache oder konjugierte Doppelbindungen sind; die Kohlenwasserstoffkette kann gegebenenfalls:

    * unterbrochen sein von einem der folgenden, als Z bezeichneten Gruppen:
      $-O-$; $-CO-$; $COO-$; $-NR_2-$; $-CO-NR_2-$; $-S-$; $-SO_2-$,
      wobei in diesen Formeln $R_2$ ein Wasserstoffatom oder einen linearen oder verzweigten Alkylrest mit 1 bis 6 Kohlenstoffatomen darstellt;
    * und/oder einen der folgenden Substituenten enthalten:
      $-OH$; $-CN$; $-N[R_2]_2$; $-COOR_2$; $-CF_3$ oder $-X$,
      wobei in diesen Formeln die Reste $R_2$, identisch oder verschieden, ein Wasserstoffatom oder einen linearen oder verzweigten Alkylrest mit 1 bis 6 Kohlenstoffatomen darstellen und X ein Halogenatom darstellt, vorzugsweise Fluor, Chlor oder Brom;

  - einen Rest vom Typ $=R_3$, bei dem $R_3$ einen Alkylidenrest mit 1 bis 6 Kohlenstoffatomen, einen Rest der Formel $=C(CN)_2$ oder einen Cycloalkyliden-oder Cycloalkenylidenrest mit 5 bis 6 Kohlenstoffatomen oder einen Benzylidenrest darstellt, der gegebenenfalls substituiert ist, vorzugsweise mit einem Halogenatom X;
  - einen linearen oder verzweigten Alkoxyrest mit 1 bis 6 Kohlenstoffatomen;
  - zwei aufeinanderfolgende Atome des Ringes können untereinander über eine Epoxy-Brücke oder über eine Brücke vom Dioxyalkylentyp mit 1 bis 4 Kohlenstoffatomen verbunden sein, vorzugsweise über Dioxymethylen-, Dioxyethylen- oder Dioxypropylenreste;
  - eine OH-Gruppe;
  - eine Gruppe $COOR_4$, wobei $R_4$ ein Wasserstoffatom oder einen Alkylrest mit 1 bis 4 Kohlenstoffatomen, vorzugsweise Methyl oder Ethyl, darstellt;
  - eine CN-Gruppe;
  - ein Halogenatom, vorzugsweise Fluor, Chlor oder Brom;
  - eine Gruppe $-CF_3$;

- R einen Rest $R_1$, eine der folgenden komplexeren, Gruppen darstellt:

  - einen gesättigten oder ungesättigten carbocyclischen Rest mit 4 bis 7 Kohlenstoffatomen, vorzugsweise einen Cyclopentyl-, Cyclohexyl-, Cyclopenten-2-yl-, Cyclopenten-3-yl-, Cyclohexen-1-yl-, Cyclohexen-2-yl- oder Cyclohexen-3-yl-Rest;

- einen Rest der Formel

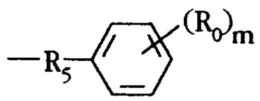

in der $R_5$ eine Valenzbindung oder einen bivalenten, linearen oder verzweigten, gesättigten oder ungesättigten Kohlenwasserstoffrest mit 1 bis 6 Kohlenstoffatomen darstellt, wie z.B. Methylen, Ethylen, Propylen, Isopropylen oder Isopropyliden, und $R_0$ die zuvor angegebene Bedeutung hat und m eine ganze Zahl von 0 bis 4 ist;

- einen Rest $-R_5-Z-R_8$, bei dem Z und $R_5$ die zuvor angegebene Bedeutung haben und $R_8$ einen linearen oder verzweigten Alkylrest mit 1 bis 6 Kohlenstoffatomen oder einen Rest der Formel

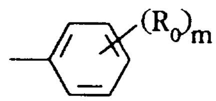

darstellt, bei dem $R_0$ und m die zuvor angegebene Bedeutung haben;

- einen Rest vom Spirotyp der Formel

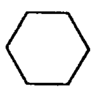
oder
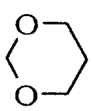
oder
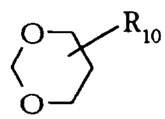

in der $R_{10}$ einen oder mehrere lineare oder verzweigte Alkylreste mit 1 bis 6 Kohlenstoffatomen bezeichnet.

**10.** Verfahren nach einem der Ansprüche 1 bis 6, dadurch gekennzeichnet, daß die Ketoverbindung der Formel (Ia) entspricht, in der A der Rest einer gesättigten, monocyclischen carbocyclischen Verbindung ist, die ein oder mehrere der folgenden Substintuenten enthält:

- einen linearen oder verzweigten Alkylrest mit 1 bis 15 Kohlenstoffatomen, vorzugsweise einen Methyl-, Ethyl-, n-Propyl-, iso-Propyl-, n-Butyl-, sec.-Butyl-, tert.-Butyl-, n-Pentyl-, iso-Pentyl-, tert.-Pentyl-, n-Hexyl- oder n-Heptylrest;
- einen Alkylrest mit 1 bis 15 Kohlenstoffatomen, der eine funktionelle Gruppe enthält, vorzugsweise eine Funktion OH, CN, $N[R_2]_2$, $COOR_2$, wobei $R_2$, identisch oder verschieden, ein Wasserstoffatom oder einen linearen oder verzweigten Alkylrest mit 1 bis 6 Kohlenstoffatomen darstellt: Die Alkylkette kann unterbrochen sein durch ein Sauerstoffatom oder eine gegebenenfalls substituierte Carbonyl-, Carboxyl- oder Aminogruppe, insbesondere kann man einen Rest der Formel $-NHCH_3$ oder $-N(CH_3)_2$, einen Rest der Formel $-CH_2-CH_2-CN$, einen Rest der Formel $-CH_2-CO-(CH_2)_4-COOH$, einen Rest der Formel $COCH(CH_3)_2$, einen Rest der Formel $-(CH_2)_6-COOH$, einen Rest der Formel $-CH_2-COOCH_3$, einen Rest der Formel $-CH_2-COOC_2H_5$, einen Rest der Formel $-CH_2-CH_2-COOCH_3$, einen Rest der Formel $-(CH_2)_6-COOCH_3$, einen Rest der Formel $-(CH_2)_6-COOC_2H_5$, einen Rest der Formel $-(CH_2)_5-COOC_2H_5$, einen Rest der Formel $-CO-CH_3$, einen Rest der Formel $-C(CH_3)_2-CO-CH_3$ und einen Rest der Formel $-CH_2-CH_2-CO-(CH_2)_4-CH_3$ nennen;
- einen linearen oder verzweigten Alkenylen- oder Alkylidenrest, der eine oder zwei Doppelbindungen mit 1 bis 15 Kohlenstoffatomen umfaßt, vorzugsweise einen Rest der Formel $-CH_2-CH=CH_2$, einen Rest der Formel $-C(CH_3)=CH_2$, einen Rest der Formel $-CH_2-CH=CH-C_2-H_5$, einen Rest der Formel $-CH_2-CH=CH-(CH_2)_2-CH_3$, einen Rest der Formel $-CH=CH-(CH_2)_4-CH_3$, einen Rest der Formel $-CH_2CH=C(CH_3)_2$ und einen Rest der Formel $-CH_2-CH=CCH_3-(CH_2)_2-CH=C(CH_3)_2$;
- einen Rest $=C(CH_3)_2$ oder $=CH-(CH_2)_2-CH_3$;
- einen linearen oder verzweigten Alkenylen- oder Alkylidenrest mit einer oder zwei Doppelbindungen mit 1 bis 15 Kohlenstoffatomen, enthaltend eine funktionelle Gruppe, vorzugsweise eine Funktion OH, CN, $NH_2$, $COOR_2$, wobei $R_2$ die zuvor angegebene Bedeutung hat: Die ungesättigte Kette kann unterbrochen sein durch

ein Sauerstoffatom, eine Carbonylgruppe oder eine Carboxylgruppe, und insbesondere kann man einen Rest der Formel $-CH_2-CH=CH-(CH_2)_3-COOH$, einen Rest der Formel $-CH=CH=C(CH_3)=CH-COOH$, einen Rest der Formel $-CH_2-CH=CH-(CH_2)_3-COOCH_3$, einen Rest der Formel $-CH=CH-C(CH_3)=CH-COOCH_3$, einen Rest der Formel $-CH=CH-CO-CH_3$, einen Rest der Formel $-CH=CH-CO-(CH_2)_4-CH_3$ oder einen Rest der Formel $-CH=CH-CHOH-(CH_2)_4-CH_3$ nennen;

- einen linearen oder verzweigten Alkoxyrest mit 1 bis 6 Kohlenstoffatomen, vorzugsweise einen Methoxy- oder Ethoxyrest;
- zwei aufeinanderfolgende Atome des Ringes können untereinander über eine Epoxy-Brücke oder über eine Brücke, wie z.B. über Methylendioxy-, Ethylendioxy- oder Propylendioxyreste, verbunden sein;
- einen Rest vom Spirotyp der folgenden Formel:

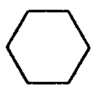
oder
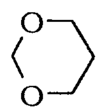
oder
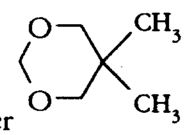

- einen Rest der Formel

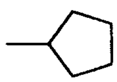
oder
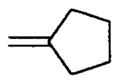
oder
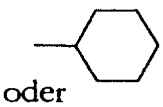
oder
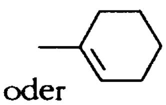

- einen Rest der Formel

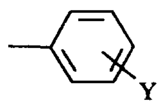
oder
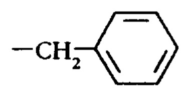
oder
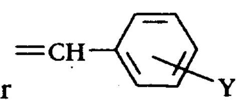

wobei Y ein Wasserstoffatom oder ein Halogenatom, vorzugsweise Fluor oder Chlor, darstellt;

- einen Rest der Formel

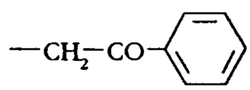
oder
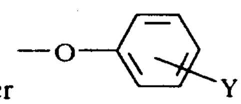

- eine OH-Gruppe;
- eine Gruppe $COOR_4$, in der $R_4$ ein Wasserstoffatom oder einen Alkylrest mit 1 bis 4 Kohlenstoffatomen, vorzugsweise einen Methyl- oder Ethylrest, bezeichnet;
- eine CN-Gruppe;
- ein Halogenatom, vorzugsweise Fluor, Chlor oder Brom.

**11.** Verfahren nach einem der Ansprüche 1 bis 6, dadurch gekennzeichnet, daß die Ketoverbindung der Formel (Ia) entspricht, in der A der Rest einer ungesättigten, monocyclischen carbocyclischen Verbindung mit einem oder mehreren der folgenden Substituenten ist:

- einem linearen oder verzweigten Alkylrest mit 1 bis 15 Kohlenstoffatomen, vorzugsweise einem Methyl-, Ethyl-, n-Propyl-, iso-Propyl-, n-Butyl-, sec.-Butyl-, tert.-Butyl-, n-Pentyl-, iso-Pentyl-, tert.-Pentyl-, n-Hexyl- oder n-Heptylrest;

- einem Alkylrest mit 1 bis 15 Kohlenstoffatomen, der eine funktionelle Gruppe enthält, vorzugsweise eine Funktion OH, CN, $N[R_2]_2$, $COOR_2$, wobei $R_2$, identisch oder verschieden, ein Wasserstoffatom oder einen linearen oder verzweigten Alkylrest mit 1 bis 6 Kohlenstoffatomen bezeichnet: Die Alkylkette kann unterbrochen sein mit einem Sauerstoffatom oder einer gegebenenfalls substituierten Carbonyl-, Carboxyl- oder Aminogruppe, und insbesondere läßt sich ein Rest der Formel $-NHCH_3$ oder $-N(CH_3)_2$, ein Rest der Formel $-CH_2-CH_2-CN$, ein Rest der Formel $CH_2-CO-(CH_2)_4-COOH$, ein Rest der Formel $COCH(CH_3)_2$, ein Rest der Formel $-(CH_2)_6-COOH$, ein Rest der Formel $-CH_2-COOCH_3$, ein Rest der Formel $-CH_2-COOC_2H_5$, ein Rest der Formel $-CH_2-CH_2-COOCH_3$, ein Rest der Formel $-(CH_2)_6-COOCH_3$, ein Rest der Formel $-(CH_2)_6-COOC_2H_5$, ein Rest der Formel $-(CH_2)_5-COOC_2H_5$, ein Rest der Formel $-CO-CH_3$, ein Rest der Formel $-C(CH_3)_2-CO-CH_3$ oder einen Rest der Formel $-CH_2-CH_2-CO-(CH_2)_4-CH_3$ nennen;
- einem linearen oder verzweigten Alkenylen- oder Alkylidenrest mit einem oder zwei Doppelbindungen mit 1 bis 15 Kohlenstoffatomen, vorzugsweise einem Rest der Formel $-CH_2-CH=CH_2$, einem Rest der Formel $-C(CH_3)=CH_2$, einem Rest der Formel $-CH_2-CH=CH-C_2H_5$, einem Rest der Formel $-CH_2-CH=CH-(CH_2)_2CH_3$, einem Rest der Formel $-CH=CH-(CH_2)_4-CH_3$, einem Rest der Formel $-CH_2-CH=C(CH_3)_2$ oder einem Rest der Formel $-CH_2-CH=CCH_3-(CH_2)_2-CH=C(CH_3)_2$;
- einem Rest $=C(CH_3)_2$ oder $=CH-(CH_2)_2-CH_3$;
- einem linearen oder verzweigten Alkenylen- oder Alkylidenrest mit einer oder zwei Doppelbindungen mit 1 bis 15 Kohlenstoffatomen mit einer funktionellen Gruppe, vorzugsweise einer Funktion OH, CN, $NH_2$ oder $COOR_2$, wobei $R_2$ die zuvor angegebene Bedeutung hat: Die ungesättigte Kette kann unterbrochen sein durch ein Sauerstoffatom oder eine Carbonyl- oder Carboxylgruppe, und insbesondere kann man nennen: einen Rest der Formel $-CH_2-CH=CH-(CH_2)_3-COOH$, einen Rest der Formel $-CH=CH-C(CH_3)=CH-COOH$, einen Rest der Formel $-CH_2-CH=CH-(CH_2)_3-COOCH_3$, einen Rest der Formel $-CH=CH-C(CH_3)=CH-COOCH_3$, einen Rest der Formel $-CH=CH-CO-CH_3$ oder einen Rest der Formel $-CH=CH-CO-(CH_2)_4-CH_3$ oder einen Rest der Formel $-CH=CH-CHOH-(CH_2)_4-CH_3$;
- einem linearen oder verzweigten Alkoxyrest mit 1 bis 6 Kohlenstoffatomen, vorzugsweise einem Methoxy- oder Ethoxyrest;
- einem Rest der Formel

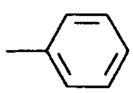

- einer OH-Gruppe;
- einer COOR-Gruppe, wobei R ein Wasserstoffatom oder einen Alkylrest mit 1 bis 4 Kohlenstoffatomen, vorzugsweise einen Methyl- oder Ethylrest, bezeichnet;
- einem Halogenatom, vorzugsweise Fluor, Chlor oder Brom.

**12.** Verfahren nach einem der Ansprüche 1 bis 5, 7 und 8, dadurch gekennzeichnet, daß die Ketoverbindung der Formel (Ia) entspricht, in der A der Rest einer polycyclischen carbocyclischen Verbindung, vorzugsweise einer bicyclischen Verbindung, ist, die zwei gesättigte Carbocyclen umfaßt, die einen oder mehrere der folgenden Substituenten enthalten:

- einen linearen oder verzweigten Alkylrest mit 1 bis 6 Kohlenstoffatomen, vorzugsweise einen Methyl- oder Isopropylrest;
- einen Rest der Formel $-CH_2Br$;
- einen linearen oder verzweigten Alkoxyrest mit 1 bis 6 Kohlenstoffatomen, vorzugsweise ein Methoxyrest;
- einen Rest der Formel $=CH_2$;
- eine OH-Gruppe;
- eine Gruppe -COOH;
- ein Halogenatom, vorzugsweise Fluor, Chlor oder Brom;
- eine $CF_3$-Gruppe.

**13.** Verfahren nach einem der Ansprüche 1 bis 5, 7 und 8, dadurch gekennzeichnet, daß die Ketoverbindung der Formel (Ia) entspricht, in der A der Rest einer bicyclischen carbocyclischen Verbindung ist, die zwei Carbocyclen

umfaßt, von denen der eine gesättigt ist und der andere ungesättigt ist, enthaltend einen oder mehreren der folgenden Substituenten:

- einen linearen oder verzweigten Alkylrest mit 1 bis 6 Kohlenstoffatomen, vorzugsweise einen Methylrest;
- einen Rest der Formel

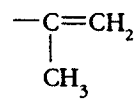

- einen Rest der Formel $-CH_2-O-CH_3$;
- ein Halogenatom, vorzugsweise Chlor.

**14.** Verfahren nach einem der Ansprüche 1 bis 5, 7 und 8, dadurch gekennzeichnet, daß die Ketoverbindung der Formel (Ia) entspricht, in der A der Rest einer polycyclischen, vorzugsweise bicyclischen, carbocyclischen Verbindung ist, die zwei ungesättigte Carbocyclen umfaßt, die einen oder mehrere Alkylreste enthalten.

**15.** Verfahren nach einem der Ansprüche 1 bis 5, 7 und 8, dadurch gekennzeichnet, daß die Ketoverbindung der Formel (Ia) entspricht, in der A der Rest einer polycyclischen, carbocyclischen Verbindung ist, die mindestens einen aromatischen Carbocyclus, vorzugsweise einen Benzolring, umfaßt, der ein oder mehrere der folgenden Substituenten enthält:

- einen linearen oder verzweigten Alkylrest mit 1 bis 6 Kohlenstoffatomen, vorzugsweise ein Methyl- oder tert.-Butylrest;
- einen Rest der Formel

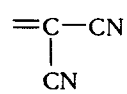

- einen linearen oder verzweigten Alkoxyrest mit 1 bis 6 Kohlenstoffatomen, vorzugsweise ein Methoxyrest;
- einen linearen oder verzweigten Alkoxyrest mit 1 bis 6 Kohlenstoffatomen, der weitere funktionelle Gruppen enthält, wie z.B. einen OH-Gruppe und/oder eine Gruppe $N[R_2]_2$, wobei $R_2$, identisch oder verschieden, ein Wasserstoffatom oder einen linearen oder verzweigten Alkylrest mit 1 bis 6 Kohlenstoffatomen, vorzugsweise einen Rest der Formel $-O-CH_2-CHOH-CH_2-NHBu$-t, darstellt;
- eine OH-Gruppe;
- eine Acylgruppe mit 2 bis 6 Kohlenstoffatomen, vorzugsweise einen Acetylrest oder einen Rest der Formel -CO-tert.-Butyl;
- eine Gruppe $-CH_2-COOH$;
- eine Gruppe $-NH_2$;
- ein Halogenatom, vorzugsweise Fluor, Chlor oder Brom.

**16.** Verfahren nach Anspruch 1, dadurch gekennzeichnet, daß die Ketoverbindung der allgemeinen Formel (Ib)

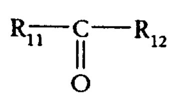 (Ib)

entspricht, wobei in der Formel (Ib):

- $R_{11}$ und $R_{12}$, identisch oder verschieden, jeweils darstellen: einen Kohlenwasserstoffrest mit 1 bis 20 Kohlenstoffatomen, der ein linearer oder verzweigter, gesättigter oder ungesättigter, acyclischer aliphatischer Rest

sein kann; einen monocyclischen oder polycyclischen, gesättigten, ungesättigten oder aromatischen, carbocyclischen oder heterocyclischen Rest; einen linearen oder verzweigten, gesättigten oder ungesättigten aliphatischen Rest, der einen cyclischen Substituenten enthält;

- mindestens einer der Reste $R_{11}$ und $R_{12}$ ein Kohlenstoffatom in $\alpha$-Position in bezug auf die Carbonylgruppe umfaßt, die mindestens ein Wasserstoffatom oder eine Esterfunktion enthält.

**17.** Verfahren nach Anspruch 16, dadurch gekennzeichnet, daß die Ketoverbindung der allgemeinen Formel (Ib) entspricht, in der $R_{11}$ und $R_{12}$ jeweils darstellen:

- einen linearen oder verzweigten, acyclischen aliphatischen Rest mit vorzugsweise 1 bis 12 Kohlenstoffatomen, der gesättigt ist oder eine bis mehrere ungesättigte Bindungen in der Kette enthält, im allgemeinen 1 bis 3 ungesättigte Bindungen, die einfache oder konjugierte Doppelbindungen sein können; die Kohlenwasserstoffkette kann gegebenenfalls durch einen der zuvor genannten Gruppen Z unterbrochen sein und/oder einen der folgenden, bereits zuvor definierten Substituenten enthalten: -OH; - CN; $N[R_2]_2$; $-COOR_2$; $-CF_3$ oder -X;
- einen linearen oder verzweigten, gesättigten oder ungesättigten, acyclischen aliphatischen Rest mit einem ringförmigen Substituenten.

**18.** Verfahren nach Anspruch 16, dadurch gekennzeichnet, daß die Ketoverbindung der allgemeinen Formel (Ib) entspricht, in der einer der Reste $R_{11}$ oder $R_{12}$ jeweils darstellt: einen gesättigten oder ungesättigten cyclischen Kohlenwasserstoffrest mit vorzugsweise 5 oder 6 Kohlenstoffatomen im Ring; einen gesättigten oder ungesättigten heterocyclischen Rest, der insbesondere 5 oder 6 Atome im Ring enthält, von denen ein oder zwei Atome Heteroatome sein können, wie z.B. Stickstoff, Schwefel oder Sauerstoff; einen kondensierten oder nichtkondensierten, monocyclischen oder polycyclischen, aromatischen Kohlenwasserstoffrest.

**19.** Verfahren nach einem der Ansprüche 1 bis 18, dadurch gekennzeichnet, daß die der Formel (Ia) entsprechende Ketoverbindung Cyclopentanon und/oder 2-Methylcyclopentanon oder 2-Methyl-2-carboxymethylcyclopentanon, 2-[(p-Chlor)benzyliden]cyclopentanon oder 2-Methyl-2-carboxymethyl-5-[(p-chlor)benzylidenlcyclopentanon ist.

**20.** Verfahren nach einem der Ansprüche 1 bis 20, dadurch gekennzeichnet, daß das Alkylierungsreagens ausgewählt ist aus organischen Estern, Alkylsulfaten und Alkoholen.

**21.** Verfahren nach Anspruch 20, dadurch gekennzeichnet, daß das Alkylierungsreagens ausgewählt ist aus organischen Estern, die der allgemeinen Formel (II) entsprechen

$$[R_a - CO - O -]_p - R_b \qquad (II)$$

in der:

- $R_a$ darstellt:
  - einen Rest $R_c$, der ein Kohlenwasserstoffrest mit 1 bis 12 Kohlenstoffatomen sein kann, der ein linearer oder verzweigter, gesättigter oder ungesättigter aliphatischer Rest sein kann, der gegebenenfalls ein Halogenatom, vorzugsweise ein Chloratom, enthält; einen monocyclischen oder polycyclischen, gesättigten, ungesättigten oder aromatischen, carbocyclischen Rest; einen linearen oder verzweigten, gesättigten oder ungesättigten, acyclischen aliphatischen Rest, der einen cyclischen Substituenten enthält;
  - einen Rest $R_d$ - O - CO - $R_f$-, in dem der Rest $R_d$ dieselbe wie zuvor für den Rest $R_c$ angegebene Bedeutung hat und der Rest $R_f$ eine Valenzbindung oder einen linearen oder verzweigten, gesättigten oder ungesättigten, aliphatischen bivalenten Rest mit mindestens 1 Kohlenstoffatom darstellt;
  - einen Rest $R_d$ - O -, in den, der Rest $R_d$ die zuvor für den Rest $R_c$ angegebene Bedeutung hat;
  - einen Rest $R_d$ - O - CO - O -, in dem der Rest $R_d$ die zuvor für den Rest $R_c$ angegebene Bedeutung hat;
- $R_b$ darstellt:
  - einen Rest $R_c$, wie zuvor definiert;
  - ein Alkali- oder Erdalkalimetall;
- $R_a$ und $R_b$ zusammen einen linearen oder verzweigten, gesättigten oder ungesättigten, aliphatischen bivalenten Rest mit mindestens 2 Kohlenstoffatomen bilden können;
- p eine ganze Zahl 1 oder 2 ist.

**22.** Verfahren nach Anspruch 20, dadurch gekennzeichnet, daß das Alkylierungsreagens ausgewählt ist aus Carbonsäureestern der Formel (IIa):

$$R_a - CO - O - R_b \qquad \text{(IIa)}$$

in der:

- $R_a$ darstellt:
  - einen linearen oder verzweigten Alkylrest mit 1 bis 6 Kohlenstoffatomen, vorzugsweise mit 1 bis 3 Kohlenstoffatomen;
  - einen Cycloalkylrest mit 5 oder 6 Kohlenstoffatomen, vorzugsweise mit 6 Kohlenstoffatomen;
  - einen Aralkylrest mit 7 bis 12 Kohlenstoffatomen;
  - einen Rest $R_c$ - O - C O - R $_d$-, in dem der Rest $R_c$ die zuvor für den Rest $R_a$ angegebene Bedeutung hat und der bivalente Test $R_d$ eine Valenzbindung oder einen Alkylenrest mit 1 bis 6 Kohlenstoffatomen darstellt;
- $R_b$ darstellt:
  - einen linearen oder verzweigten Alkylrest mit 1 bis 6 Kohlenstoffatomen;
  - einen Cycloalkylrest mit 5 oder 6 Kohlenstoffatomen;
- $R_a$ und $R_b$ zusammen einen Alkylenrest mit 2 bis 4 Kohlenstoffatomen bilden können.

**23.** Verfahren nach Anspruch 20, dadurch gekennzeichnet, daß das Alkylierungsreagens ausgewählt ist aus Carbonaten der allgemeinen Formel (IIb):

$$[R_a - O - CO - O -]_p - R_b \qquad \text{(IIb)}$$

in der:

- $R_a$ darstellt:
  - einen linearen oder verzweigten Alkylrest mit 1 bis 6 Kohlenstoffatomen, der gegebenenfalls ein Halogenatom, vorzugsweise ein Chloratom, enthält;
  - einen Cycloalkylrest mit 5 bis 6 Koluenstoffatomen;
  - einen Arylrest mit 6 bis 12 Kohlenstoffatomen;
  - einen Rest $R_d$ - O - CO -, in dem $R_d$ einen linearen oder verzweigten Alkylrest mit 1 bis 6 Kohlenstoffatomen oder einen Cycloalkylrest mit 5 bis 6 Kohlenstoffatomen darstellt;
- $R_b$ darstellt:
  - einen linearen oder verzweigten Alkylrest mit 1 bis 6 Kohlenstoffatomen;
  - einen Cycloalkylrest mit 5 oder 6 Kohlenstoffatomen;
  - ein Alkali- oder Erdalkalimetall, vorzugsweise Natrium- oder Kalium, wobei p = 1 oder p = 2 gilt, wenn $R_b$ ein Erdalkalimetall darstellt;
- $R_a$ und $R_b$ zusammen einen Alkylenrest mit 2 bis 6 Kohlenstoffatomen bilden können.

**24.** Verfahren nach Anspruch 20, dadurch gekennzeichnet, daß das Alkylierungsreagens ausgewählt ist aus Dialkylsulfaten der Formel $R_g$-O-$SO_2$-O-$R_h$, wobei in dieser Formel $R_g$ und $R_h$, identisch oder verschieden, jeweils einen linearen oder verzweigten Alkylrest mit 1 bis 6 Kohlenstoffatomen bezeichnen.

**25.** Verfahren nach Anspruch 20, dadurch gekennzeichnet, daß das Alkylierungsreagens ausgewählt ist aus Alkoholen der Formel (IIc):

$$R_i - OH \qquad \text{(IIc)}$$

wobei in dieser Formel $R_i$ darstellt: einen Kohlenwasserstoffrest mit 1 bis 12 Kohlenstoffatomen, der ein linearer

oder verzweigter, gesättigter oder ungesättigter, acyclischer aliphatischer Rest sein kann; einen monocyclischen oder polycyclischen, gesättigten oder ungesättigten cycloaliphatischen Rest; einen linearen oder verzweigten, gesättigten oder ungesättigten aliphatischen Rest, der einen ringförmigen Substituenten enthalten kann.

**26.** Verfahren nach einem der Ansprüche 20 bis 25, dadurch gekennzeichnet, daß das Alkylierungsreagens ausgewählt ist aus: Dimethylsulfat; Dimethylcarbonat, Chlormethylmethylcarbonat; Methylacetat oder Ethylacetat; Methanol oder Ethanol.

**27.** Verfahren nach einem der Ansprüche 1 bis 26, dadurch gekennzeichnet, daß der Katalysator ein Metall- oder Ammoniumphosphat ist, wie z.B. ein Dihydrogenphosphat, ein Monohydrogenphosphat, ein Phosphat, ein kondensiertes Phosphat, vorzugsweise ein Pyrophosphat oder ein Polyphosphat.

**28.** Verfahren nach Anspruch 27, dadurch gekennzeichnet, daß der Katalysator ein Orthophosphat eines Elementes der Gruppe 1a, 2a, 3a oder 3b, vorzugsweise der Gruppe 3a, ist.

**29.** Verfahren nach einem der Ansprüche 27 und 28, dadurch gekennzeichnet, daß der eingesetzte Katalysator ausgewählt ist aus Natrium- oder Kaliumphosphaten, -pyrophosphaten, -tripolyphosphaten oder -pentapolyphosphaten.

**30.** Verfahren nach einem der Ansprüche 27 und 28, dadurch gekennzeichnet, daß der erfindungsgemäße Katalysator der folgenden allgemeinen Formel entspricht:

$$MPO_4 \qquad (III)$$

in der:

- M eine trivalente (dreiwertige) Seltene Erde, nämlich ein Lanthanid, mit einer Atomzahl zwischen 57 und 71 und Yttrium sowie Scandium darstellt.

**31.** Verfahren nach einem der Ansprüche 27 und 28, dadurch gekennzeichnet, daß der erfindungsgemäße Katalysator der folgenden allgemeinen Formel entspricht:

$$MPO_4\,(Im)_p \qquad (IIIa)$$

in der:

- M eine trivalente (dreiwertige) Seltene Erde $M_3$ oder eine Mischung aus mindestens einer trivalenten Seltenen Erde $M_3$ und einem Element darstellt, das ausgewählt ist aus Alkalimetallen $M_1$ und Erdalkalimetallen $M_2$ mit der folgenden Beziehung:

$$M = \alpha M_1^{\ +} + \beta M_2^{\ ++} + \gamma M_3^{\ 3+} \text{ und } \alpha + 2\beta + 3\gamma = 3$$

- *Im* einer basischen Verbindung entspricht, die aus einem Erdalkalimetall und vorzugsweise aus einem Alkalimetall und deren Mischungen besteht, das oder die mit einem Gegenanion zur Herstellung der elektrischen Neutralität verbunden sind;
- $\alpha$ ein Koeffizient zwischen 0 und 3, vorzugsweise größer als 0,01 und höchstens 0,5, insbesondere ein Koeffizient zwischen 0,05 und 0,2, ist;
- $\beta$ ein Koeffizient zwischen 0 und 3/2, vorzugsweise zwischen 0 und 1/3 oder auch 1 $\pm$ 0,1 ist;
- $\gamma$ ein Koeffizient zwischen 0 und 1, vorzugsweise von mindestens gleich 1/3, insbesondere von 1/2, ist;
- p kleiner als 0,5 ist und vorzugsweise zwischen 0,04 und 0,25 liegt.

**32.** Verfahren nach Anspruch 31, dadurch gekennzeichnet, daß der erfindungsgemäße Katalysator der Formel (IIIa) entspricht, in der:

- $M_1$ ausgewählt ist aus der Gruppe von Elementen der Gruppe 1a und ihren Mischungen, vorzugsweise aus Alkalimetallen wie Lithium, Natrium, Kalium, Rubidium und Cäsium;
- $M_2$ ausgewählt ist aus Elementen der Gruppe 2a und ihren Mischungen, vorzugsweise aus Erdalkalimetallen wie Beryllium, Magnesium, Calcium, Strontium und Barium;

- $M_3$ ausgewählt ist aus der Gruppe der trivalenten (dreiwertigen) Seltenen Erden, ausgewählt aus Linthaniden, Yttrium, Scandium und ihren Mischungen, vorzugsweise aus Lanthaniden wie Lanthan, Cer, Praseodym, Neodym, Samarium, Europium, Gadolinium, Terbium, Dysprosium, Holmium, Erbium, Thulium, Ytterbium und Lutecium.

**33.** Verfahren nach einem der Ansprüche 30 bis 32, dadurch gekennzeichnet, daß der erfindungsgemäße Katalysator ein Orthophosphat einer trivalenten (dreiwertigen) Seltenen Erde ist, vorzugsweise einer cerhaltigen Seltenen Erde, die gegebenenfalls mit einem Alkali- oder Erdalkalimetall dotiert ist.

**34.** Verfahren nach einem der Ansprüche 30 bis 33, dadurch gekennzeichnet, daß der erfindungsgemäße Katalysator der Formel (IIIa) entspricht, in der M Lanthan, Cer und Samarium ist, dotiert mit einem Alkalimetall, vorzugsweise Cäsium.

**35.** Verfahren nach einem der Ansprüche 30 bis 34, dadurch gekennzeichnet, daß der erfindungsgemäße Katalysator einen solchen Gehalt an dem Dotier-Reagenz aufweist, daß der Gewichtsprozentgehalt dieses Dotiermittels, bezogen auf das Phosphat der trivalenten Seltenen Erde, zwischen 0 und 25 %, vorzugsweise zwischen 3 und 15 %, liegt.

**36.** Verfahren nach Anspruch 30, dadurch gekennzeichnet, daß die Ausgangsketoverbindung, die mit einem Alkylierungsreagens zur Reaktion gebracht wird, mindestens ein Wasserstoffatom in $\alpha$-Position zur Carbonylgruppe aufweist.

**37.** Verfahren nach einem der Ansprüche 27 bis 36, dadurch gekennzeichnet, daß die Ausgangsketoverbindung, die zur Reaktion gebracht wird, mindestens eine Esterfunktion in $\alpha$-Position zur Carbonylgruppe aufweist.

**38.** Verfahren nach einem der Ansprüche 30 bis 37, dadurch gekennzeichnet, daß die Menge an Alkylierungsreagenz derart gewählt ist, daß das Verhältnis zwischen der Molanzahl an Alkylierungsreagenz und der Zahl der Wasserstoffatome, die durch eine Alkylgruppe einer Ketoverbindung ersetzt werden, zwischen 0,5 und 500, vorzugsweise zwischen 1 und 10, variiert.

**39.** Verfahren nach einem der Ansprüche 1 bis 38, dadurch gekennzeichnet, daß die stündliche Gewichtsproduktivität des Katalysators zwischen 0,1 und 20 $h^{-1}$, vorzugsweise zwischen 1 und 5 $h^{-1}$, liegt.

**40.** Verfahren nach einem der Ansprüche 1 bis 39, dadurch gekennzeichnet, daß die Reaktionstemperatur für die C-Alkylierung zwischen 80 °C und 500 °C, vorzugsweise zwischen 200 °C und 350 °C, liegt.

**41.** Verfahren nach einem der Ansprüche 1 bis 40, dadurch gekennzeichnet, daß die Reaktion in der Gasphase durchgeführt wird.

**42.** Verfahren nach einem der Ansprüche 1 bis 40, dadurch gekennzeichnet, daß die Reaktion in flüssiger Phase, gegebenenfalls in Gegenwart eines organischen Lösemittels, durchgeführt wird.